# EUROPEAN PATENT APPLICATION

(11) **EP 4 789 685 A1**
(43) Date of publication of application: **12.08.2026**
(21) Application number: 25382084.9
(22) Date of filing: 05.02.2025
(51) Int. Cl.: A61K 47/68, A61P 35/00

(54) **ANTI-ENDOGLIN ANTIBODY-DRUG CONJUGATES**

(71) Applicant: Oncomatryx Biopharma, S.L., 48160 Derio (Vizcaya) (ES)
(72) Inventor: The designation of the inventor has not yet been filed
(74) Representative: Mewburn Ellis LLP

(57) **Abstract**

The present invention relates to an antibody-drug conjugate having the Formula (II), or a pharmaceutically acceptable salt or solvate thereof: 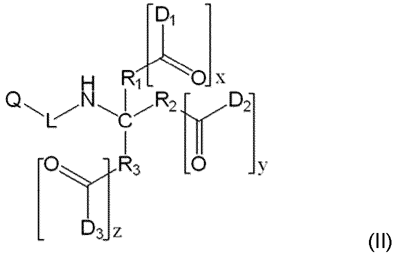 wherein Q is an antibody that selectively binds Endoglin; L is a linker; R₁, R₂, and R₃ are each independently a bridging group; D₁, D₂, and D₃ are each independently Eribulin; and each of x, y, and z is independently 0 or 1, with the provision that at least one of x, y and z is 1.

## Description

### Field of the Invention

The present invention relates to antibody-drug conjugates (ADCs) that target Endoglin (ENG), and to their use in a method of treating a disease, e.g. in the treatment of cancer.

### Background

Malignant epithelial tumours are the main cancer-related cause of human death. These solid tumours frequently exhibit significant stromal reactions such as the so-called "desmoplastic stroma" or "reactive stroma", which represents 20-60% of total tumour mass and is characterized by the existence of large numbers of stromal cells and dense extracellular matrix (ECM). Studies have indicated the tumour-promoting roles of stromal cells, as exemplified by vascular cells, immune cells, fibroblasts, myofibroblasts, adipocytes and bone marrow-derived progenitors *(1-6).* Considerable numbers of cancer-associated fibroblasts (CAFs) are frequently observed within tumour-associated stroma of various human cancers, including breast, lung, colon, and pancreas carcinomas *(7,8).* Interacting coordinately with the different components of the stroma, CAFs have the ability to promote neoangiogenesis and tumour growth; CAFs have also been shown as crucial for the development of aggressive tumours and tumour invasiveness during cancer progression *(9-18);* CAFs facilitate the spreading and infiltration of tumour cells in distant organs, thus contributing to formation of metastases. Importantly, the relevance of stromal cells to the failure of systemic drug delivery to tumours and to the development of drug resistance has also been indicated *(19-23).*

Monoclonal antibody (MAb) - based drugs represent a great promise in the fight against cancer. This is because they allow the treatment to be aimed at a molecular level in a precise and specific way. These advantages, together with their commercial appeal (short development times, restricted competence and being easily exportable to other cancer types once they have been approved), have pushed many pharmaceutical companies to invest heavily in the development of new antibody-based molecules, as well as in the in-licensing of new molecules or technologies from biotech companies.

However, despite the clinical success of therapeutic antibodies, naked MAbs targeting cell surface tumour antigens rarely present sufficient efficacy on their own. To increase the low activity of the MAbs, novel strategies are focusing on binding them to toxic molecules. Plant and bacterial toxins as well as small chemotherapeutic molecules can be good candidates, since they are very potent and active in very small quantities.

The field of Antibody-Drug conjugates (ADCs) for the treatment of cancer has recently experienced a growing development activity by pharmaceutical companies, due to the technological advances performed during the last years, aimed at solving the problems initially presented about immunogenicity, undesirable toxicity, production, half-life and resistance.

Immunoconjugates are made of a human, humanized or chimeric recombinant antibody, covalently linked to a cytotoxic drug. The main goal of such a structure is joining the power of small cytotoxic molecules (300 to 1000Da) and the high specificity of tumour-associated antigen targeted (TAA) MAbs.

The Ab must be very selective to reach the antigen, whose expression must be restricted in normal cells. The Ab also must be internalized efficiently into the cancerous cells.

The cytotoxic agent selected as the effector moiety must kill cells only after internalization and release into the cell cytoplasm. The most commonly used payloads in ADCs are DNA-harming drugs such as calicheamicins, duocarmicins, or microtubule-targeting compounds like auristatins and maitansinoids. The Ab-cytotoxic linkers are designed to be stable systemically and to release the cytotoxic within the target cells.

TAAs are cell membrane proteins that are often overexpressed in diseased tissues or at least expressed sufficiently to facilitate the internalization-activated cytotoxicity. Ideally the antigen presents a restricted expression in normal tissues with a low or absent expression in vital organs. On top of this, the tumour antigen must be recognized selectively and with high affinity by an Ab.

Recent studies suggest that therapeutic agents designed to inhibit TGF-β signaling pathway at the tumour-stroma interphase could prevent cancer progression, improving prognosis and treatment. TGF-β co-receptor family has emerged as a target for cancer treatments acting on the tumour or on its neovasculature. Endoglin (ENG, CD105), an accessory protein of the type II TGF-β receptor complex, is part of this family and presents the following characteristics:
- Type I homodimer membrane protein
- Cell surface angiogenesis and neovascularisation-associated protein in many cancer types
- Overexpressed in tumour microvasculature cells, specifically in angiogenic areas of tumour
- No expression in normal tissue endothelium
- Breast metastasis-correlated plasma levels
- Internalization
- Optimal accessibility from bloodstream.

Anti-ENG antibodies (e.g. scFvs) have been reported and their application in tumour stroma targeting strategies described. Specific binding, cell internalization and anti-tumoural effects of Doxorubicin-loaded, anti-ENG immunoliposomes have been reported *in vitro,* using ENG+ cells, and *in vivo* in mice. ENG-targeted conjugates of anti-ENG antibodies and ricin or native nigrin b have been evaluated in mouse tumour models (24-33). WO 2015/118031 discloses an anti-ENG antibody-drug conjugate, which may otherwise be referred to as OMTX703, wherein the drug comprises a cytolysin or a Nigrin-b A-chain, and *in vivo* anti-tumour activity of this ADC.

Despite these advances, there remains a need for further and improved ADCs for the treatment of tumours, including epithelial tumours.

The present invention has been devised in light of the above considerations.

### Summary of the Invention

Broadly, the present invention relates to antibody-drug conjugates, wherein the antibody selectively binds endoglin (ENG) and is conjugated to a drug comprising Eribulin. The present inventors have found that anti-ENG antibody-drug conjugates as described herein exhibit highly specific binding and improved *in vitro* and *in vivo* cytotoxicity compared to other anti-ENG antibody-drug conjugates.

Accordingly, in a first aspect the present invention provides an antibody-drug conjugate having the Formula (II), 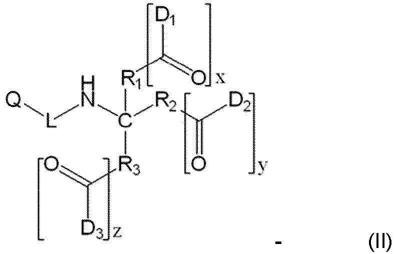
or a pharmaceutically acceptable salt or solvate thereof,
wherein:
   Q is an antibody that selectively binds Endoglin;
      L is a linker;
      each of D1, D2 and D3 is independently Eribulin;
      each of x, y and z is independently 0 or 1, with the provision that at least one of x, y and z is 1;
      each of R₁, R₂, and R₃ is independently selected from: a bond, hydrogen, 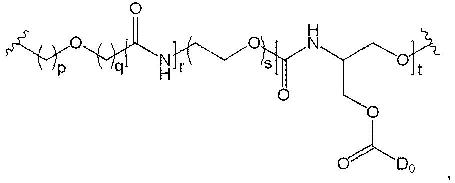 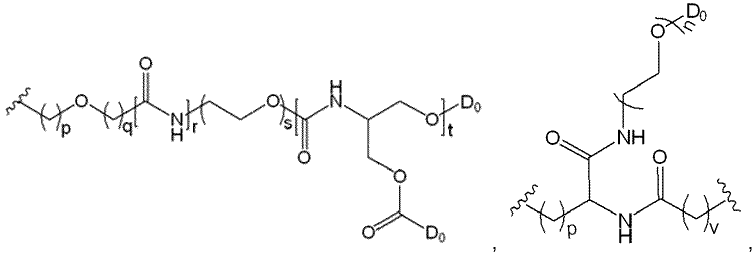 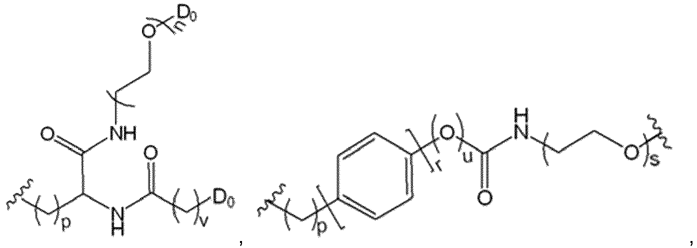 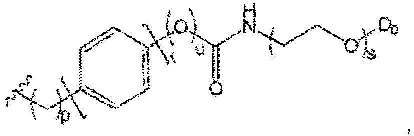 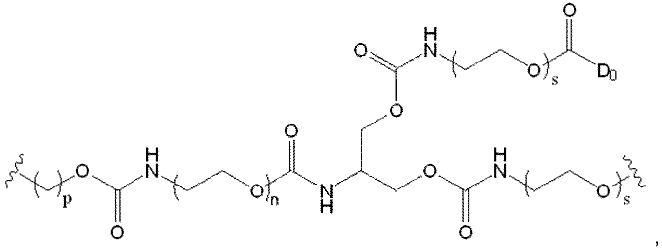 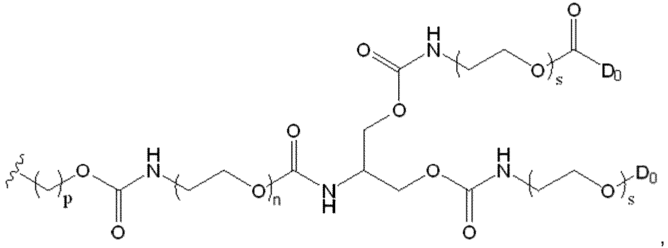 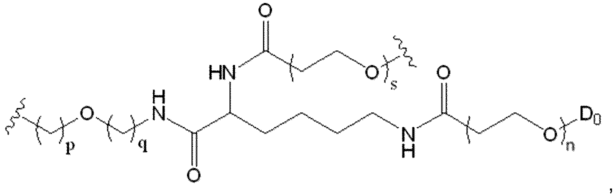 and 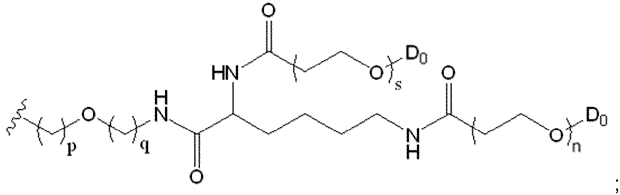 wherein p is 1, 2, or 3; q is 0, 1, or 2; r is 0 or 1; s is an integer from 0 to 37; t is 0 or 1; u is 0 or 1; v is 1, 2, or 3; and n is an integer from 1 to 37; or
      x and y are 0 and R₁ and R₂ together form =O and R₃ is 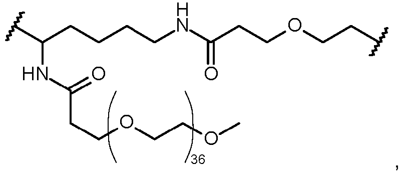
      D₀ is methyl, propargyl, or Eribulin;
      with a provision that at least one of R₁, R₂, and R₃ is not hydrogen;
      if q, r, s, and t are 0, then x+y+z>1;
      if x is 0, then R₁ is selected from: hydrogen, 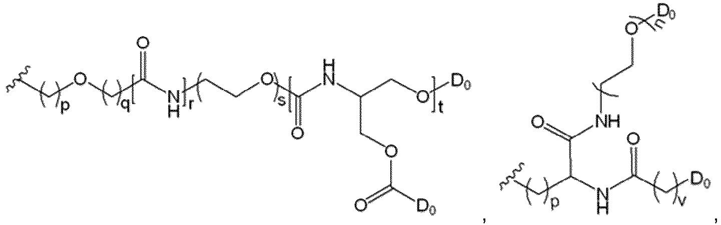 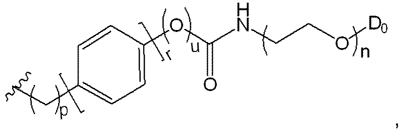 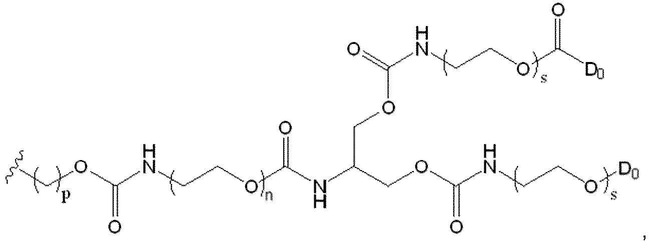 and 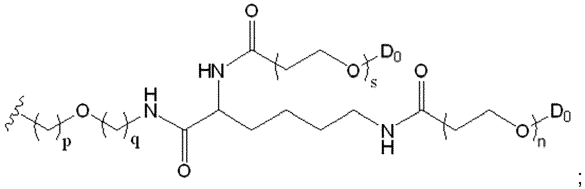
      if y is 0, then R₂ is selected from: hydrogen, 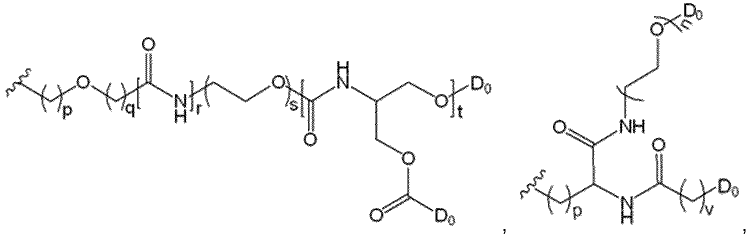 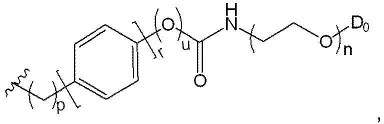 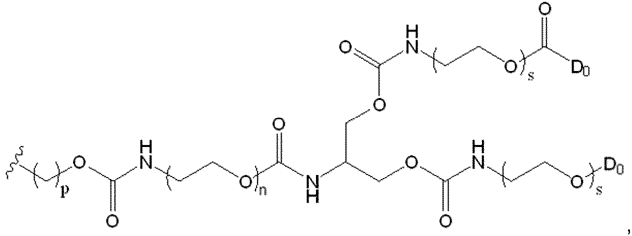 and 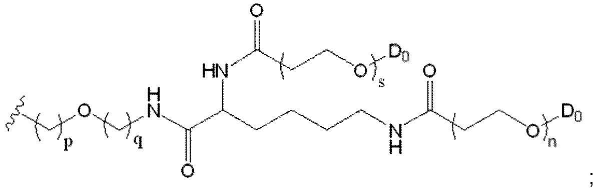
      if z is 0, then R₃ is selected from: hydrogen, 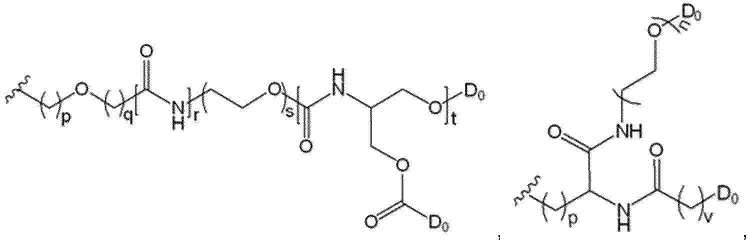 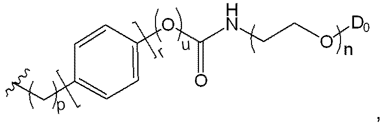 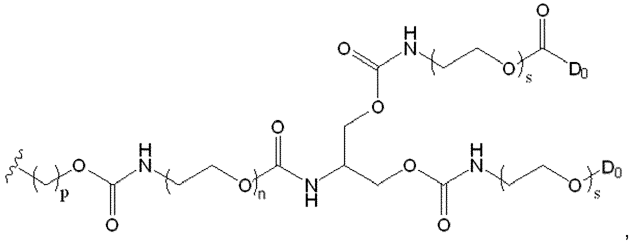 and 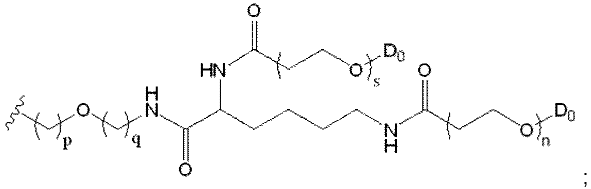
      optionally, if x+y+z=1 and each of R₁, R₂, and R₃ independently is 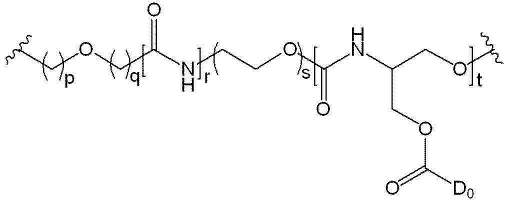 or 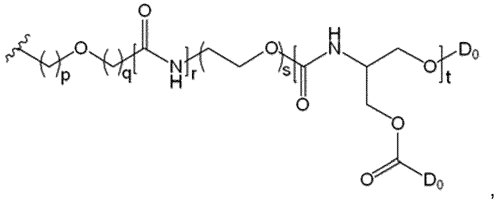 then p is not 1; and
   optionally, if r, s, and t are 0 and each of R₁, R₂, and R₃ independently is 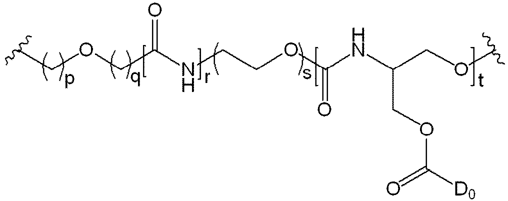 or 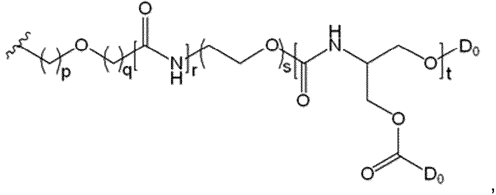 then x+y+z>1.

In some embodiments Q is a monoclonal antibody or binding fragment thereof that selectively binds to an extracellular region of human Endoglin. In some embodiments Q comprises heavy chain complementarity determining regions 1-3 (CDRH1-3) and light chain complementarity determining regions 1-3 (CDRL1-3) having the following amino acid sequences:
(i) CDRH1: SEQ ID NO: 7 or a variant thereof having up to 1 or 2 amino acid substitutions compared with the sequence of SEQ ID NO: 7;
(ii) CDRH2: SEQ ID NO: 8 or a variant thereof having up to 1 or 2 amino acid substitutions compared with the sequence of SEQ ID NO: 8;
(iii) CDRH3: SEQ ID NO: 9 or a variant thereof having up to 1 or 2 amino acid substitutions compared with the sequence of SEQ ID NO: 9;
(iv) CDRL1: SEQ ID NO: 10 or a variant thereof having up to 1 or 2 amino acid substitutions compared with the sequence of SEQ ID NO: 10;
(v) CDRL2: SEQ ID NO: 11 or a variant thereof having up to 1 or 2 amino acid substitutions compared with the sequence of SEQ ID NO: 11; and
(vi) CDRL3: SEQ ID NO: 12 or a variant thereof having up to 1 or 2 amino acid substitutions compared with the sequence of SEQ ID NO: 12.

In some embodiments, CDRH1-3 comprise the amino acid sequences of SEQ ID NOS: 7-9, respectively and CDRL1-3 comprise the amino acid sequences of SEQ ID NOS: 10-12, respectively.

In some embodiments, Q comprises a heavy chain variable region (VH) comprising an amino acid sequence having at least 90%, 95% or 99% sequence identity with the full-length sequence of SEQ ID NO: 5.

In some embodiments, Q comprises a heavy chain variable region (VH) comprising the amino acid sequence of SEQ ID NO: 5.

In some embodiments, Q comprises a light chain variable region (VL) comprising an amino acid sequence having at least 90%, 95% or 99% sequence identity with the full-length sequence of SEQ ID NO: 6. In particular, Q may comprise a light chain variable region (VL) comprising the amino acid sequence of SEQ ID NO: 6.

In some embodiments, Q comprises a heavy chain comprising an amino acid sequence having at least 90%, 95% or 99% sequence identity with the full-length sequence of SEQ ID NO: 3. In particular, Q may comprise a heavy chain comprising the amino acid sequence of SEQ ID NO: 3.

In some embodiments, Q comprises a light chain comprising an amino acid sequence having at least 90%, 95% or 99% sequence identity with the full-length sequence of SEQ ID NO: 4. In particular, Q may comprise a light chain comprising the amino acid sequence of SEQ ID NO: 4.

In some embodiments, Q is a monoclonal antibody or binding fragment thereof that selectively binds to an extracellular region of murine Endoglin. Conjugates that target murine Endoglin find particular use in pre-clinical testing, e.g., employing well-characterised murine models of various cancers. In particular, Q may comprise heavy chain complementarity determining regions 1-3 (CDRH1-3) and light chain complementarity determining regions 1-3 (CDRL1-3) having the following amino acid sequences:
(i) CDRH1: SEQ ID NO: 19 or a variant thereof having up to 1 or 2 amino acid substitutions compared with the sequence of SEQ ID NO: 19;
(ii) CDRH2: SEQ ID NO: 20 or a variant thereof having up to 1 or 2 amino acid substitutions compared with the sequence of SEQ ID NO: 20;
(iii) CDRH3: SEQ ID NO: 21 or a variant thereof having up to 1 or 2 amino acid substitutions compared with the sequence of SEQ ID NO: 21;
(iv) CDRL1: SEQ ID NO: 22 or a variant thereof having up to 1 or 2 amino acid substitutions compared with the sequence of SEQ ID NO: 22;
(v) CDRL2: SEQ ID NO: 23 or a variant thereof having up to 1 or 2 amino acid substitutions compared with the sequence of SEQ ID NO: 23; and
(vi) CDRL3: SEQ ID NO: 24 or a variant thereof having up to 1 or 2 amino acid substitutions compared with the sequence of SEQ ID NO: 24. For example, CDRH1-3 may comprise the amino acid sequences of SEQ ID NOS: 19-21, respectively and CDRL1-3 may comprise the amino acid sequences of SEQ ID NOS: 22-24, respectively.

In some embodiments, Q comprises a heavy chain variable region (VH) comprising an amino acid sequence having at least 90%, 95% or 99% sequence identity with the full-length sequence of SEQ ID NO: 17, e.g. Q may comprise a heavy chain variable region (VH) comprising the amino acid sequence of SEQ ID NO: 17.

In some embodiments, Q comprises a light chain variable region (VL) comprising an amino acid sequence having at least 90%, 95% or 99% sequence identity with the full-length sequence of SEQ ID NO: 18, e.g., Q may comprise a light chain variable region (VL) comprising the amino acid sequence of SEQ ID NO: 18.

In some embodiments, Q comprises: a) a heavy chain variable region (VH) comprising an amino acid sequence having at least 90%, 95% or 99% sequence identity with the full-length sequence of SEQ ID NO: 17; optionally a heavy chain variable region (VH) comprising the amino acid sequence of SEQ ID NO: 17; and/or b) a light chain variable region (VL) comprising an amino acid sequence having at least 90%, 95% or 99% sequence identity with the full-length sequence of SEQ ID NO: 18; optionally a light chain variable region (VL) comprising the amino acid sequence of SEQ ID NO: 18.

In some embodiments, Q comprises a heavy chain comprising an amino acid sequence having at least 90%, 95% or 99% or even 100% sequence identity with the full-length sequence of SEQ ID NO: 15.

In some embodiments, Q comprises a light chain comprising an amino acid sequence having at least 90%, 95% or 99% or even 100% sequence identity with the full-length sequence of SEQ ID NO: 16.

In some embodiments, Q comprises: a) a heavy chain comprising an amino acid sequence having at least 90%, 95% or 99% or even 100% sequence identity with the full-length sequence of SEQ ID NO: 15; and/or b) a light chain comprising an amino acid sequence having at least 90%, 95% or 99% or even 100% sequence identity with the full-length sequence of SEQ ID NO: 16.

In a second aspect the present invention provides a conjugate as defined in accordance with the first aspect of the invention for use in a method of treating a disease in a mammalian subject. In some embodiments, the disease is a cancer or an inflammatory disease. In some embodiments, the disease is a cancer, arthritis, or fibrosis. In some embodiments, the disease is a cancer or arthritis (e.g. rheumatoid arthritis). In some embodiments, the disease is a cancer or fibrosis. In some embodiments, the disease is arthritis (e.g. rheumatoid arthritis) or fibrosis.

According to a third aspect, the present invention provides a conjugate as defined in accordance with the first aspect of the invention for use in a method of treating cancer in a mammalian subject. The cancer may comprise a solid tumour. Alternatively, the cancer may comprise blood cancer. In particular, the cancer may comprise gastric cancer, pancreatic cancer, Ewing sarcoma, breast cancer, melanoma, lung cancer, head & neck cancer, ovarian cancer, bladder cancer or colon cancer.

In some embodiments the cancer is an Endoglin-positive cancer (including any of the above-described cancers) that expresses Endoglin on one or more cancer cells and/or on one or more cells forming part of the tumour microenvironment, in particular, one or more cells of the stroma. The cancer may be any stage. In particular, the cancer may be a metastatic cancer. In particular, the cancer may be gastric cancer that expresses Endoglin.

In a fourth aspect, the present invention provides a conjugate as defined in accordance with the first aspect of the invention for use in a method of treating an inflammatory disease in a mammalian subject. The inflammatory disease may be fibrotic (e.g., fibrosis, e.g., pulmonary fibrosis), autoimmune (e.g., rheumatoid arthritis), allergic (e.g., asthma), infectious (e.g., tuberculosis), rheumatic (e.g., ankylosing spondylitis), gastrointestinal (e.g., Crohn's disease), dermatological (e.g., psoriasis), neurological (e.g., multiple sclerosis), pulmonary (e.g., chronic obstructive pulmonary disease), cardiovascular (e.g., myocarditis), renal (e.g., glomerulonephritis), endocrine (e.g., Hashimoto's thyroiditis), or systemic (e.g., systemic lupus erythematosus). In some embodiments, the inflammatory disease is fibrosis. The fibrosis may be of the liver, lung, heart and/or colon. In some embodiments, the inflammatory disease is arthritis, for example rheumatoid arthritis. In some embodiments, the inflammatory disease is selected from: fibrosis, arthritis, psoriasis, systemic lupus erythematosus (SLE), inflammatory bowel diseases such as Crohn's disease and ulcerative colitis, asthma, atopic dermatitis, ankylosing spondylitis, multiple sclerosis, Hashimoto's thyroiditis, and sarcoidosis. In some embodiments, the fibrosis is pulmonary fibrosis (e.g., idiopathic pulmonary fibrosis), liver fibrosis, or a cancer-associated fibrosis.

In some embodiments of any of the aspects, the conjugate is for simultaneous, sequential or separate administration with one or more other antitumour drugs. The one or more other antitumour drugs may comprise a cytotoxic chemotherapeutic agent or an anti-angiogenic agent or an immunotherapeutic agent. In some embodiments the one or more other antitumour drugs comprise Gemcitabine, Nabpaclitaxel (Abraxane^{®}), bevacizumab, itraconazole, carboxyamidotriazole, an anti-PD-1 molecule (for example, nivolumab, tislelizumab, or pembrolizumab) or an anti-PD-L1 molecule.

In a further aspect the present invention provides a method of treating a disease in a mammalian subject, the method comprising administering a therapeutically effective amount of a conjugate as defined in accordance with the first aspect of the invention to the subject in need thereof. The disease may comprise cancer. The cancer may be as defined above in relation to the third aspect. The disease may comprise an inflammatory disease. The inflammatory disease may be as defined above in relation to the fourth aspect.

In a further aspect, the present invention provides the use of a conjugate as defined in accordance with the first aspect in the manufacture of a medicament for the treatment of a disease in a mammalian subject. The disease may comprise cancer. The cancer may be as defined above in relation to the third aspect. The disease may comprise an inflammatory disease. The inflammatory disease may be as defined above in relation to the fourth aspect.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:

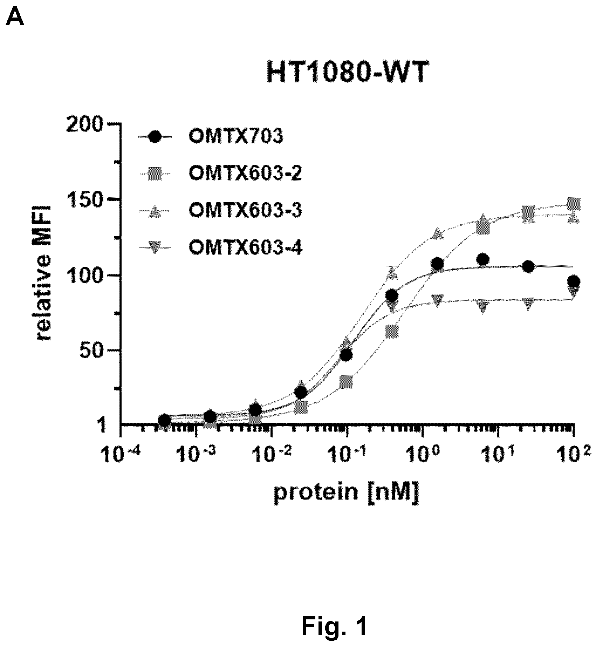

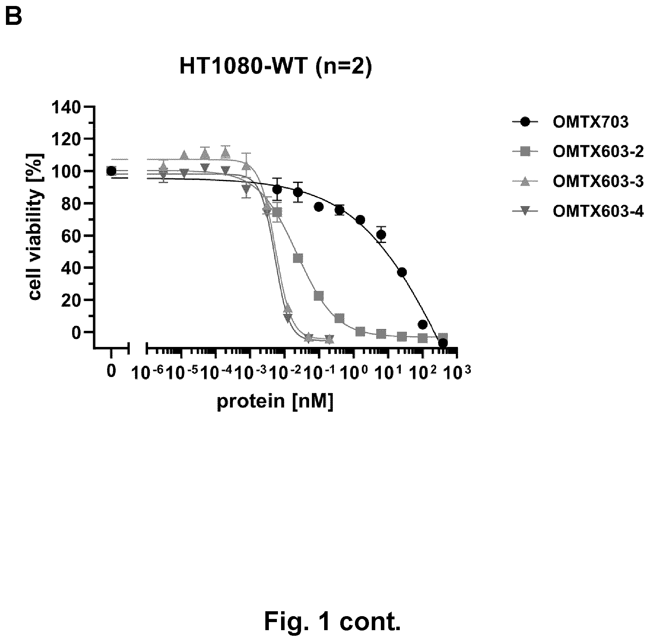

**Figure 1****. *In vitro* cell binding and cytotoxicity assays for three conjugates of the present invention: OMTX603-2, OMTX603-3 and OMTX603-4.** (A) *In vitro* cell binding assay for OMTX603-2, OMTX603-3 and OMTX603-4 compared to OMTX703, an endoglin-targeted antibody conjugated to a cytolysin payload. (B) In vitro cytotoxicity assay for OMTX603-2, OMTX603-3 and OMTX603-4 compared to OMTX703, an endoglin-targeted antibody conjugated to a cytolysin payload. The cell viability assay used HT1080-WT cells.

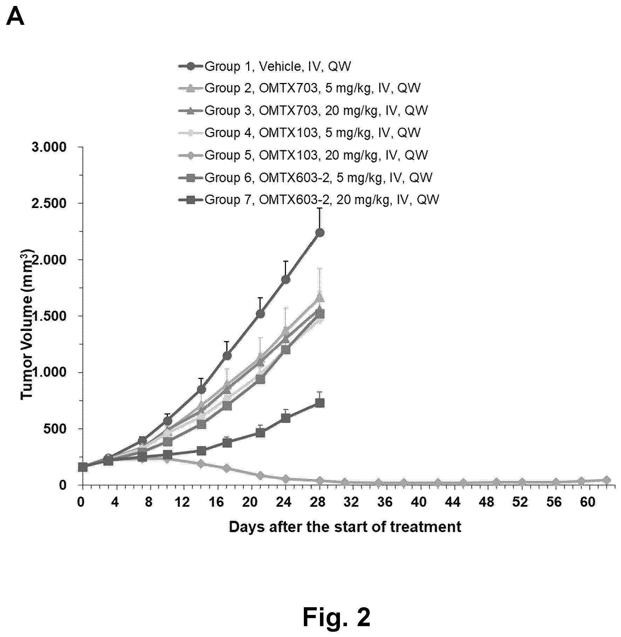

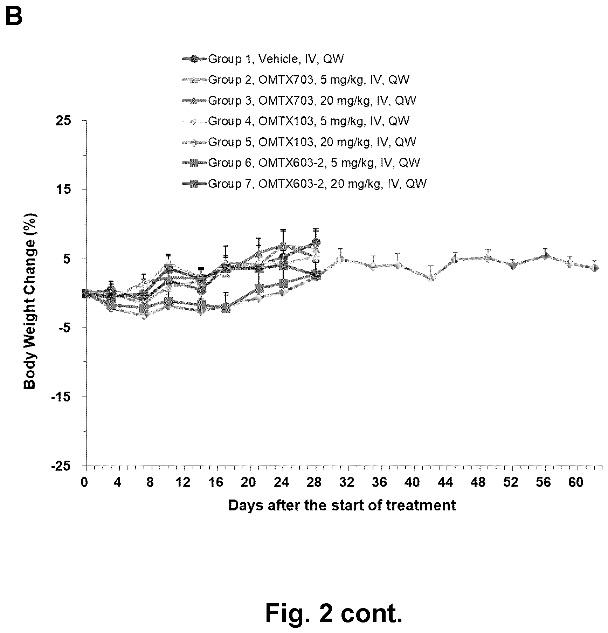

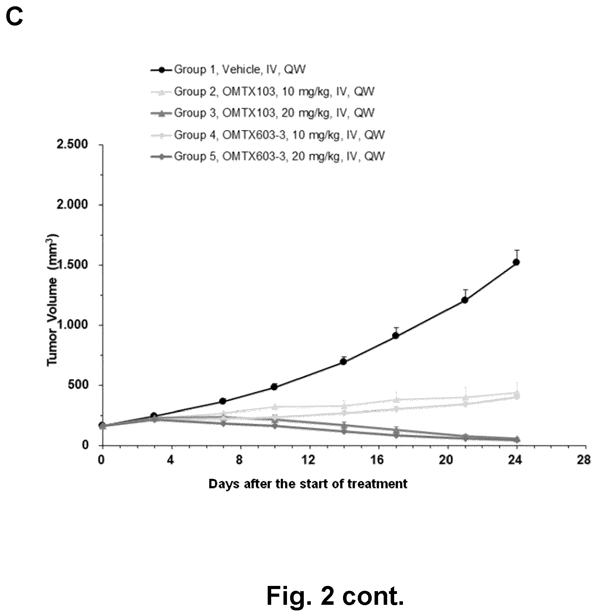

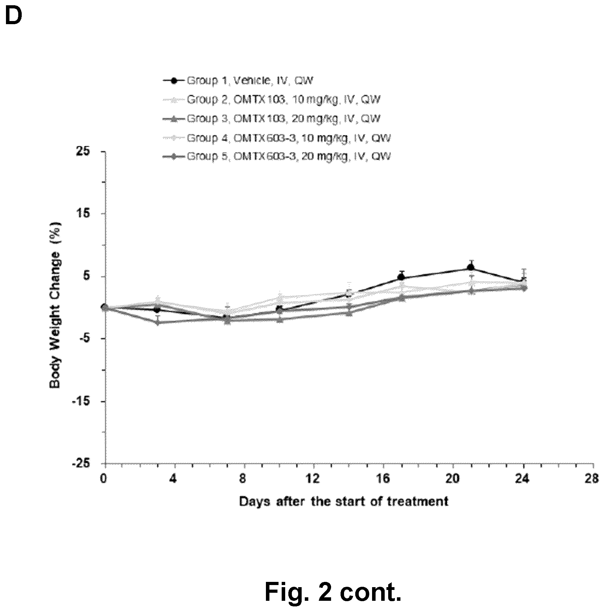

**Figure 2****. *In vivo* anti-tumoral efficacy of OMTX603-2 and OMTX603-3 in a gastric cancer PDX model, ST-02-0318, with high expression of endoglin.** (A) Change in tumour volume following administration of OMTX603-2 at different weekly i.v. doses of 5 and 20 mg/kg compared to administration of OMTX703 and OMTX103 at 5 and 20 mg/kg, and a vehicle control. (B) Body weight change of mice in (A). (C) Change in tumour volume following administration of OMTX603-3 at different weekly i.v. doses of 10 and 20 mg/kg compared to OMTX103 at weekly i.v. doses of 10 and 20 mg/kg and a vehicle control. (D) Body weight change of mice in (C).

### Detailed Description of the Invention

In describing the present invention, the following terms will be employed, and are intended to be defined as indicated below.

### Endoglin

As used "Endoglin" may be an Endoglin protein of any mammalian species. In some embodiments Endoglin is human Endoglin (also known as CD105, ENG or END), the amino acid sequence of which is disclosed at UniProt accession No. P17813 (Version 154, dated 13 November 2013) (SEQ ID NO: 25). In some embodiments, a molecule that binds Endoglin (e.g. an antibody molecule or a conjugate thereof) may bind to a region of the extracellular domain of Endoglin. The extracellular domain of human Endoglin comprises residues 26-561 of the full-length human Endoglin protein. In some embodiments Endoglin is murine Endoglin (also known as CD105, MJ7/18 antigen, ENG or END), the amino acid sequence of which is disclosed at UniProt accession No. Q63961 (Version 104, dated 13 November 2013) (SEQ ID NO: 26). The extracellular domain of murine Endoglin comprises residues 27-581 of the full-length murine Endoglin protein.

### Conjugate

As used herein "conjugate" includes the resultant structure formed by linking molecules and specifically includes antibody-drug conjugates (ADCs).

### Selectively binds

The terms selectively binds and selective binding refer to binding of an antibody, or binding fragment thereof, to a predetermined molecule (e.g. an antigen) in a specific manner. For example, the antibody, or binding fragment thereof, may bind to Endoglin, e.g. an extracellular portion thereof, with an affinity of at least about 1x10⁷M⁻¹, for example as measured at 25°C (e.g. measured by surface plasmon resonance(SPR), such as Biacore SPR). The antibody or binding fragment thereof may bind to the predetermined molecule (e.g. Endoglin) with an affinity that is at least two-fold greater (e.g. five-fold or ten-fold greater) than its affinity for binding to a molecule other than the predetermined molecule.

### Antibody molecule

As used herein with reference to all aspects of the invention, the term "antibody" or "antibody molecule" includes any immunoglobulin whether natural or partly or wholly synthetically produced. The term "antibody" or "antibody molecule" includes monoclonal antibodies (mAb) and polyclonal antibodies (including polyclonal antisera). Antibodies may be intact or fragments derived from full antibodies (see below). Antibodies may be human antibodies, humanised antibodies or antibodies of non-human origin. "Monoclonal antibodies" are homogeneous, highly specific antibody populations directed against a single antigenic site or "determinant" of the target molecule. "Polyclonal antibodies" include heterogeneous antibody populations that are directed against different antigenic determinants of the target molecule. The term "antiserum" or "antisera" refers to blood serum containing antibodies obtained from immunized animals.

It has been shown that fragments of a whole antibody can perform the function of binding antigens. Thus reference to antibody herein, and with reference to the methods and compositions of the invention, covers a full antibody and also covers any polypeptide or protein comprising an antibody binding fragment. Examples of binding fragments are (i) the Fab fragment consisting of V_{L}, V_{H}, C_{L} and C_{H}1 domains; (ii) the Fd fragment consisting of the V_{H} and C_{H}1 domains; (iii) the Fv fragment consisting of the V_{L} and V_{H} domains of a single antibody; (iv) the dAb fragment which consists of a V_{H} domain; (v) isolated CDR regions; (vi) F(ab')₂ fragments, a bivalent fragment comprising two linked Fab fragments (vii) single chain Fv molecules (scFv), wherein a V_{H} domain and a V_{L} domain are linked by a peptide linker which allows the two domains to associate to form an antigen binding site; (viii) bispecific single chain Fv dimers (WO 93/11161) and (ix) "diabodies", multivalent or multispecific fragments constructed by gene fusion (WO94/13804; 58). Fv, scFv or diabody molecules may be stabilised by the incorporation of disulphide bridges linking the VH and VL domains. Minibodies comprising a scFv joined to a CH3 domain may also be made.

In relation to an antibody molecule, the term "selectively binds" may be used herein to refer to the situation in which one member of a specific binding pair will not show any significant binding to molecules other than its specific binding partner(s). The term is also applicable where e.g. an antigen-binding site is specific for a particular epitope that is carried by a number of antigens, in which case the specific binding member carrying the antigen-binding site will be able to bind to the various antigens carrying the epitope. The antibody may be a fully human antibody.

Q may comprise a human monoclonal antibody.

For example, Q may comprise a human monoclonal antibody that:
(i) selectively binds human Endoglin and which comprises a heavy chain comprising the amino acid sequence of SEQ ID NO: 3 and a light chain comprising the amino acid sequence of SEQ ID NO: 4; or
(ii) selectively binds murine Endoglin and which comprises a heavy chain comprising the amino acid sequence of SEQ ID NO: 15 and a light chain comprising the amino acid sequence of SEQ ID NO: 16.

In some embodiments, Q comprises or consists of a human monoclonal antibody that selectively binds human Endoglin and which comprises a heavy chain comprising the amino acid sequence of SEQ ID NO: 3 and a light chain comprising the amino acid sequence of SEQ ID NO: 4.

### Pharmaceutical compositions

The conjugates of the present invention may be comprised in pharmaceutical compositions with a pharmaceutically acceptable excipient.

A pharmaceutically acceptable excipient may be a compound or a combination of compounds entering into a pharmaceutical composition which does not provoke secondary reactions and which allows, for example, facilitation of the administration of the conjugate, an increase in its lifespan and/or in its efficacy in the body or an increase in its solubility in solution. These pharmaceutically acceptable vehicles are well known and will be adapted by the person skilled in the art as a function of the mode of administration of the conjugate.

In some embodiments, conjugates of the present invention may be provided in a lyophilised form for reconstitution prior to administration. For example, lyophilised conjugates may be re-constituted in sterile water and mixed with saline prior to administration to an individual.

Conjugates of the present invention will usually be administered in the form of a pharmaceutical composition, which may comprise at least one component in addition to the conjugate. Thus, pharmaceutical compositions may comprise, in addition to the conjugate, a pharmaceutically acceptable excipient, carrier, buffer, stabilizer or other materials well known to those skilled in the art. Such materials should be non-toxic and should not interfere with the efficacy of the conjugate. The precise nature of the carrier or other material will depend on the route of administration, which may be by bolus, infusion, injection or any other suitable route, as discussed below.

For intra-venous administration, e.g. by injection, the pharmaceutical composition comprising the conjugate may be in the form of a parenterally acceptable aqueous solution which is pyrogen-free and has suitable pH, isotonicity and stability. Those of relevant skill in the art are well able to prepare suitable solutions using, for example, isotonic vehicles, such as Sodium Chloride Injection, Ringer's Injection, Lactated Ringer's Injection. Preservatives, stabilizers, buffers, antioxidants and/or other additives may be employed as required including buffers such as phosphate, citrate and other organic acids; antioxidants, such as ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3'-pentanol; and m-cresol); low molecular weight polypeptides; proteins, such as serum albumin, gelatin or immunoglobulins; hydrophilic polymers, such as polyvinylpyrrolidone; amino acids, such as glycine, glutamine, asparagines, histidine, arginine, or lysine; monosaccharides, disaccharides and other carbohydrates including glucose, mannose or dextrins; chelating agents, such as EDTA; sugars, such as sucrose, mannitol, trehalose or sorbitol; salt-forming counter-ions, such as sodium; metal complexes (e.g. Zn-protein complexes); and/or non-ionic surfactants, such as TWEEN^{™}, PLURONICS^{™} or polyethylene glycol (PEG).

### Subject

The subject may be a human, a companion animal (e.g. a dog or cat), a laboratory animal (e.g. a mouse, rat, rabbit, pig or non-human primate), a domestic or farm animal (e.g. a pig, cow, horse or sheep). Preferably, the subject is a human. In some embodiments, the subject is a human diagnosed with or classified as being at risk of developing a cancer, e.g., an epithelial tumour, a solid tumour or a blood cancer. In certain embodiments the subject may be a laboratory animal, e.g., a mouse model of a cancer.

### Cancer

The anti-ENG conjugates described herein find particular use in a method of treatment of cancer in a mammalian subject. The cancer may comprise blood cancer. The cancer may comprise a solid tumour. The cancer may comprise gastric cancer, pancreatic cancer, breast cancer, melanoma, Ewing sarcoma, lung cancer, head & neck cancer, ovarian cancer, bladder cancer or colon cancer. The cancer may be an Endoglin positive cancer (including any of the above-described cancers) that expresses Endoglin on one or more cancer cells and/or on one or more cells forming part of the tumour microenvironment, in particular, one or more cells of the stroma. The cancer may be any stage. In particular, the cancer may be a metastatic cancer. In particular, the cancer may be gastric cancer that expresses Endoglin. The cancer may have been previously treated with one or more of chemotherapy, surgical resection, radiation therapy, immunotherapy or cell therapy. For example, the cancer may be a cancer in which a first line therapy has failed to prevent progression to a more advanced stage.

### Dosages

Multiple doses of the conjugate may be provided. One or more, or each, of the doses may be accompanied by simultaneous, sequential or separate administration of another anti-cancer agent.

In some embodiments the time interval between administration steps performed sequentially is one of at least 12 hours, 24 hours, 36 hours, 48 hours, 72 hours, 4 days, 5 days, 7 days, 10 days, 14 days, 18 days, 21 days, or 28 days, or 1, 2, 3, 4, 5, or 6 months. The conjugate may be administered at a dose equivalent to between 5 mg/kg IV and 50 mg/kg IV, e.g. between 10 mg/kg and 30 mg/kg or at approximately 20 mg/kg IV. Referring to Fig. 2C, the tumour volume of the Group 5 mice (treated with a conjugate of the present invention at a dose of 20 mg/kg IV per week, was drastically reduced and maintained at a near zero volume for a substantial period of time (e.g. from around day 28 to beyond day 40). Without wishing to be bound by any particular theory, the inventors presently consider that a human dose equivalent to the murine dose of 20 mg/kg IV per week may show particular efficacy in the treatment of gastric cancer, among other forms of cancer.

In embodiments where administration steps comprise plural, separate introductions of material into a subject, time intervals provided herein may be between the final introduction of one administration step, and the first introduction of the subsequent administration step.

Preferably, the conjugate of the present invention, or a pharmaceutical composition comprising the conjugate according to the present invention is administered via injection. Such administration may be both via infusion (continuous) or bolus (discrete) administration. The method of administration via injection may be, for example, subcutaneous, intradermal, intramuscular, intravenous, intraarterial, intracardiac, intrathecal, intraspinal, intracapsular, subcapsular, intra-orbital, intraperitoneal, intratracheal, subcuticular, intraarticular, subarachnoid, and intra-sternal injection. Preferably, the administration is by intravenous infusion or intravenous injection (bolus administration). More preferably, the administration is by intravenous infusion.

Administration of the articles according to the present disclosure is preferably in a 'therapeutically-effective' or 'prophylactically-effective' amount, this being sufficient to show therapeutic/prophylactic benefit to the subject. The actual amount administered, and rate and time-course of administration, will depend on the nature and severity of the disease/condition and the particular article administered. Prescription of treatment, e.g. decisions on dosage *etc.*, is within the responsibility of general practitioners and other medical doctors, and typically takes account of the disease/disorder to be treated, the condition of the individual subject, the site of delivery, the method of administration and other factors known to practitioners. Examples of the techniques and protocols mentioned above can be found in Remington's 'The Science and Practice of Pharmacy' (ed. A. Adejare), 23rd Edition (2020), Academic Press.

### Combination therapy

The conjugate or composition of the present invention may be administered or be for administration simultaneously, sequentially or separately with one or more other anti-cancer agents. For example, the conjugate of the present invention, or a pharmaceutical composition comprising the conjugate according to the present invention, may be administered simultaneously, sequentially or separately with a chemotherapeutic agent or an immune checkpoint inhibitor. Examples of immune checkpoint inhibitors (ICls) (such as monoclonal antibodies) include PD-1 inhibitors, PD-L1 inhibitors, CTLA-4 inhibitors, LAG-3 inhibitors, and combinations thereof. In particular, an ICI may be selected from: ipilimumab, tislelizumab, tremelimumab, pembrolizumab, nivolumab, cemiplimab, atezolizumab, avelumab, durvalumab and relatlimab.

### Sequence identity

As used herein, 'sequence identity' refers to the percent of nucleotides/amino acid residues in a subject sequence that are identical to nucleotides/amino acid residues in a reference sequence, after aligning the sequences and, if necessary, introducing gaps, to achieve the maximum percent sequence identity between the sequences. Pairwise and multiple sequence alignment for the purposes of determining percent sequence identity between two or more amino acid or nucleic acid sequences can be achieved in various ways known to a person of skill in the art, for instance, using publicly available computer software such as ClustalOmega (Söding, J. 2005, Bioinformatics 21, 951-960), T-coffee (Notredame et al. 2000, J. Mol. Biol. (2000) 302, 205-217), Kalign (Lassmann and Sonnhammer 2005, BMC Bioinformatics, 6(298)) and MAFFT (Katoh and Standley 2013, Molecular Biology and Evolution, 30(4) 772-780) software. When using such software, the default parameters, e.g. for gap penalty and extension penalty, are preferably used.

### Isomers

Certain compounds of the invention may exist in one or more particular geometric, optical, enantiomeric, diasteriomeric, epimeric, atropic, stereoisomeric, tautomeric, conformational, or anomeric forms, including but not limited to, cis- and trans-forms; E- and Z-forms; c-, t-, and r- forms; endo- and exo-forms; R-, S-, and meso-forms; D- and L-forms; d- and I-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms").

The term "chiral" refers to a molecule with a non-superimposable mirror image. The term "achiral" refers to molecules which are superimposable on their mirror image partner.

The term "stereoisomers" refers to compounds which have the same molecular formula and sequence of bonded atoms, but differ in the three-dimensional orientation of their atoms in space.

"Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds of the invention may contain asymmetric or chiral centres, and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention, even if only one isomer is shown for a given compound or structure. Many organic compounds exist in optically active forms, i.e., they have the ability to rotate the plane of plane-polarized light. In describing an optically active compound, the prefixes D and L, or *R* and S, are used to denote the absolute configuration of the molecule about its chiral centre(s). The prefixes d and I or (+) and (-) are employed to designate the sign of rotation of plane-polarized light by the compound, with (-) or I meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there is no stereospecificity in a chemical reaction or process. The terms "racemic mixture" and "racemate" refer to an equimolar mixture of two enantiomeric species, devoid of optical activity.

"Enantiomerically enriched form" refers to a sample of a chiral substance whose enantiomeric ratio is greater than 50:50 but less than 100:0.

Note that, except as discussed below for tautomeric forms, specifically excluded from the term "isomers", as used herein, are structural (or constitutional) isomers (i.e. isomers which differ in the connections between atoms rather than merely by the position of atoms in space). For example, a reference to a methoxy group, -OCH₃, is not to be construed as a reference to its structural isomer, a hydroxymethyl group, -CH₂OH. Similarly, a reference to ortho-chlorophenyl is not to be construed as a reference to its structural isomer, meta-chlorophenyl. However, a reference to a class of structures may well include structurally isomeric forms falling within that class (e.g. C₁₋₇ alkyl includes n-propyl and iso-propyl; butyl includes n-, iso-, sec-, and tert-butyl; methoxyphenyl includes ortho-, meta-, and para-methoxyphenyl).

The above exclusion does not pertain to tautomeric forms, for example, keto-, enol-, and enolate-forms, as in, for example, the following tautomeric pairs: keto/enol (illustrated below), imine/enamine, amide/imino alcohol, amidine/enediamine, nitroso/oxime, thioketone/enethiol, and N-nitroso/hyroxyazo.

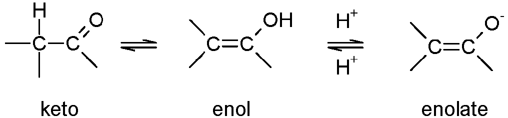

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons. Accordingly, the compounds according to the present invention include all tautomeric forms even if only one is shown.

The compounds according to the present invention include all isotopic forms even if only one is shown, i.e., compounds with one or more isotopic substitutions. For example, H may be in any isotopic form, including ¹H, ²H (D), and ³H (T); C may be in any isotopic form, including ¹²C, ¹³C, and ¹⁴C; O may be in any isotopic form, including ¹⁶O and ¹⁸O; and the like.

Examples of isotopes that can be incorporated into compounds of the invention include isotopes of hydrogen, carbon, nitrogen, oxygen, phosphorous, fluorine, chlorine and iodine, such as, but not limited to ²H (deuterium, D), ³H (tritium), ¹¹C, ¹³C, ¹⁴C, ¹⁵N, ¹⁸F, ³¹P, ³²P, ³⁵S, ³⁶Cl, and ¹²⁵I. Various isotopically labelled compounds of the present invention, for example those into which radioactive isotopes such as ³H, ¹³C, and ¹⁴C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays, or in radioactive treatment of patients. Deuterium labelled or substituted therapeutic compounds of the invention may have improved DMPK (drug metabolism and pharmacokinetics) properties, relating to distribution, metabolism, and excretion (ADME). Substitution with heavier isotopes such as deuterium may afford certain therapeutic advantages resulting from greater metabolic stability, for example increased in vivo half-life or reduced dosage requirements. An ¹⁸F labelled compound may be useful for PET or SPECT studies. Isotopically labelled compounds of this invention and prodrugs thereof can generally be prepared by carrying out the procedures disclosed in the schemes or in the examples and preparations described below by substituting a readily available isotopically labelled reagent for a non-isotopically labelled reagent. Further, substitution with heavier isotopes, particularly deuterium (i.e., ²H or D) may afford certain therapeutic advantages resulting from greater metabolic stability, for example increased in vivo half-life or reduced dosage requirements or an improvement in therapeutic index. It is understood that deuterium in this context is regarded as a substituent. The concentration of such a heavier isotope, specifically deuterium, may be defined by an isotopic enrichment factor. In the compounds of this invention any atom not specifically designated as a particular isotope is meant to represent any stable isotope of that atom.

Unless otherwise specified, a reference to a particular compound includes all such isomeric forms, including (wholly or partially) racemic and other mixtures thereof. Methods for the preparation (e.g. asymmetric synthesis) and separation (e.g. fractional crystallisation and chromatographic means) of such isomeric forms are either known in the art or are readily obtained by adapting the methods taught herein, or known methods, in a known manner.

### Conjugates According to Formula (II)

Provided herein is an antibody-drug conjugate having a structure of Formula (II), or a pharmaceutically acceptable salt or solvate thereof: 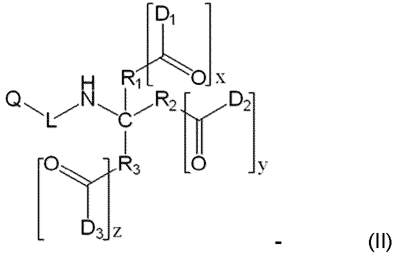
or a pharmaceutically acceptable salt or solvate thereof,
wherein:
   Q is an antibody that selectively binds Endoglin;
      L is a linker;
      each of D1, D2 and D3 is independently Eribulin;
      each of x, y and z is independently 0 or 1, with the provision that at least one of x, y and z is 1;
      each of R₁, R₂, and R₃ is independently selected from: a bond, hydrogen, 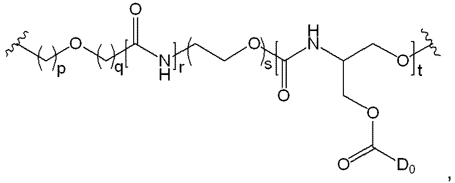 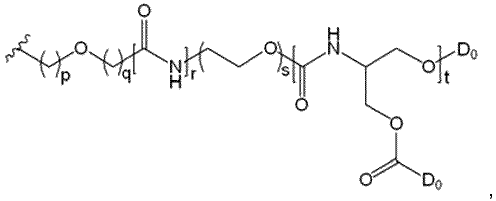 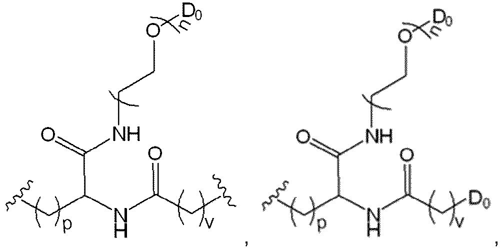 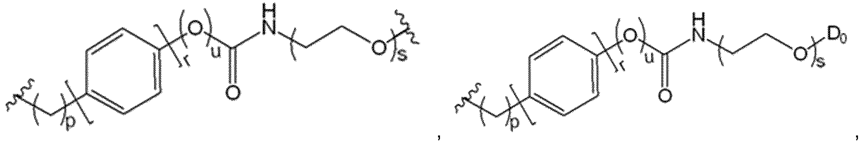 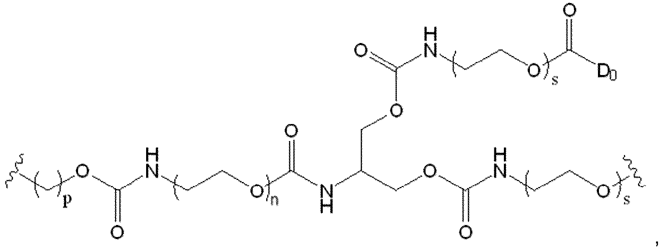 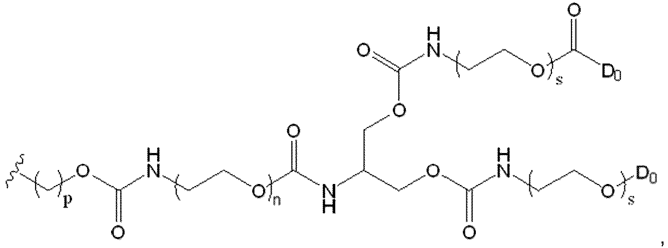 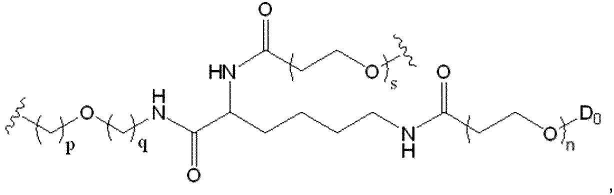 and 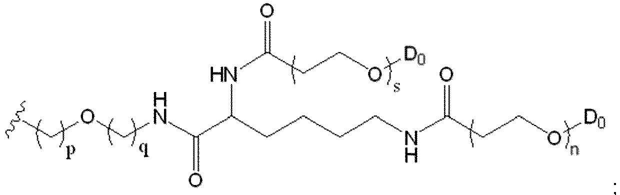 wherein p is 1, 2, or 3; q is 0, 1, or 2; r is 0 or 1; s is an integer from 0 to 37; t is 0 or 1; u is 0 or 1; v is 1, 2, or 3; and n is an integer from 1 to 37; or
      x and y are 0 and R₁ and R₂ together form =O and R₃ is 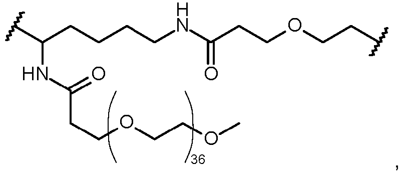
      D₀ is methyl, propargyl, or Eribulin;
      with a provision that at least one of R₁, R₂, and R₃ is not hydrogen;
      if q, r, s, and t are 0, then x+y+z>1;
      if x is 0, then R₁ is selected from: hydrogen, 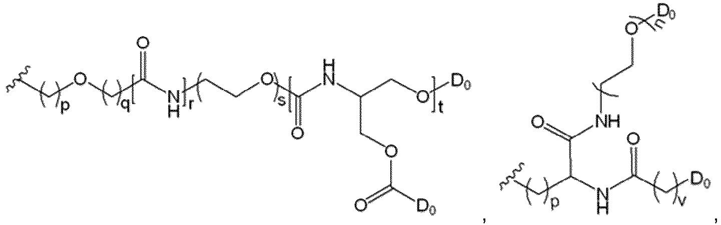 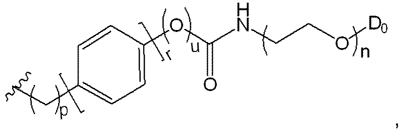 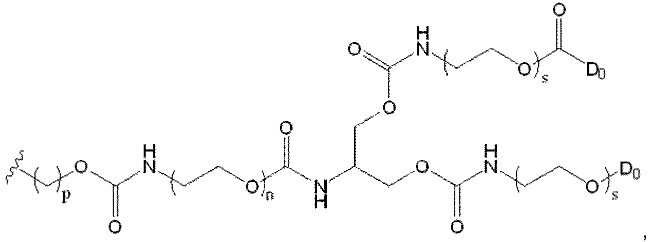 and 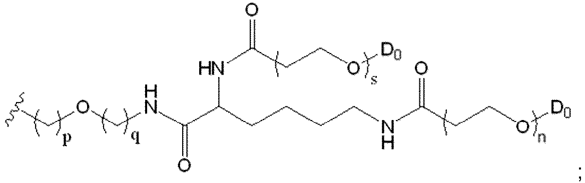
      if y is 0, then R₂ is selected from: hydrogen, 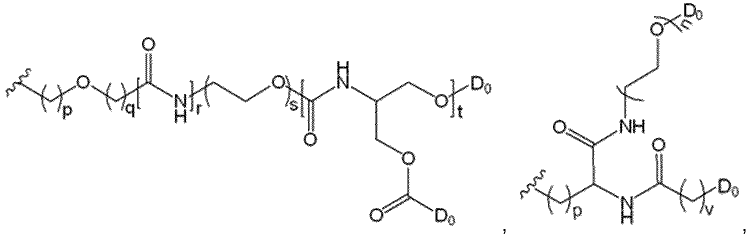 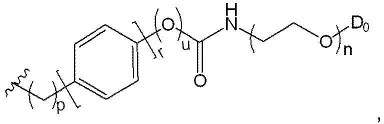 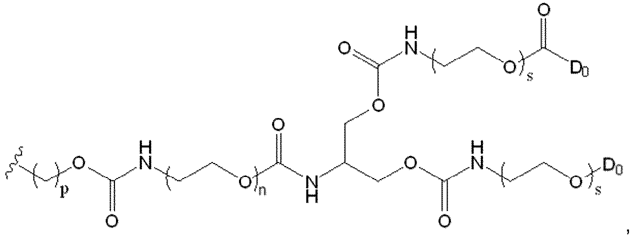 and 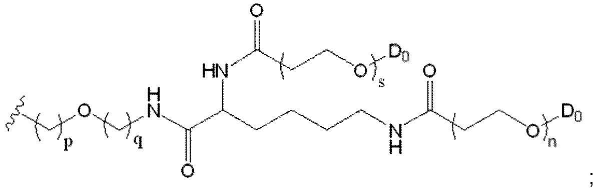
      if z is 0, then R₃ is selected from: hydrogen, 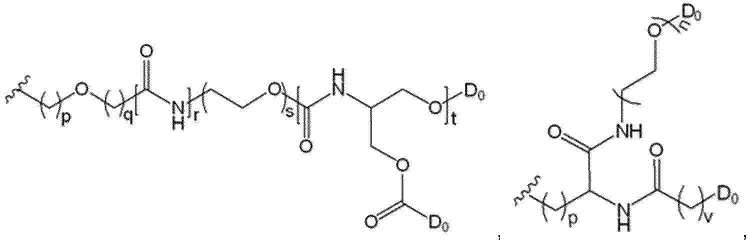 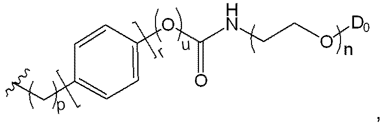 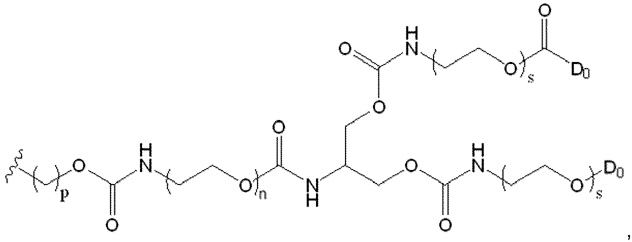 and 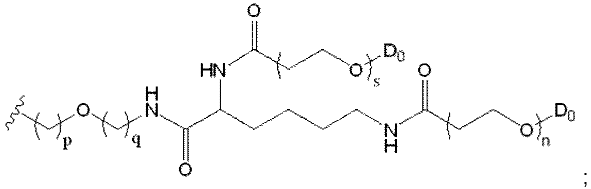
      optionally, if x+y+z=1 and each of R₁, R₂, and R₃ independently is 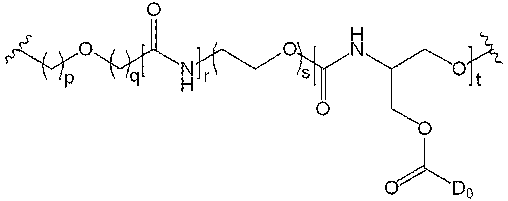 or 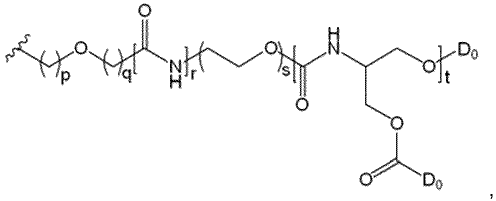 then p is not 1; and
   optionally, if r, s, and t are 0 and each of R₁, R₂, and R₃ independently is 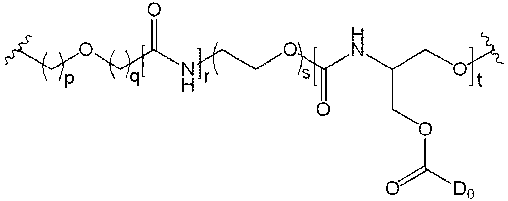 or 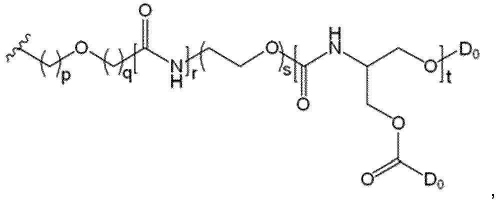 then x+y+z>1.

Also provided herein is an antibody-drug conjugate having a structure of Formula (II), or a pharmaceutically acceptable salt or solvate thereof: 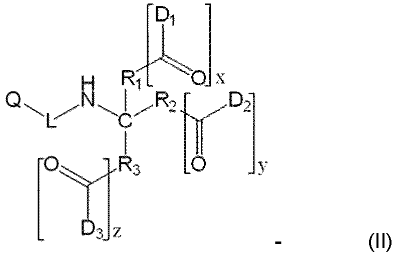
or a pharmaceutically acceptable salt or solvate thereof,
wherein:
   **Q** is an antibody that selectively binds Endoglin;
   L is a linker;
   each of D1, D2 and D3 (where present) is independently Eribulin;
   each of x, y and z is independently 0 or 1, with the provision that at least one of x, y and z is 1;
   each of R₁, R₂, and R₃ is independently selected from: a bond, hydrogen, 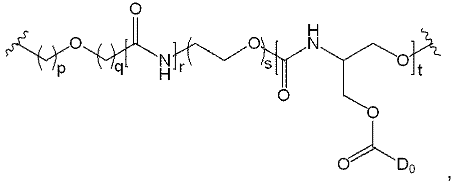 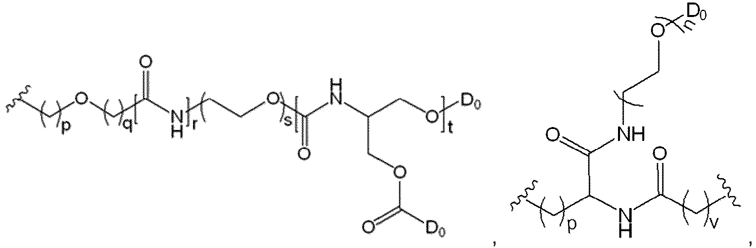 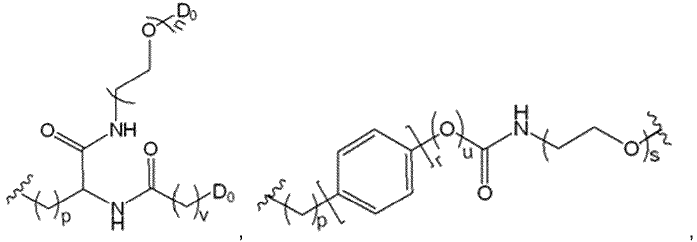 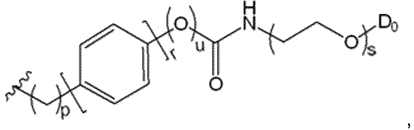 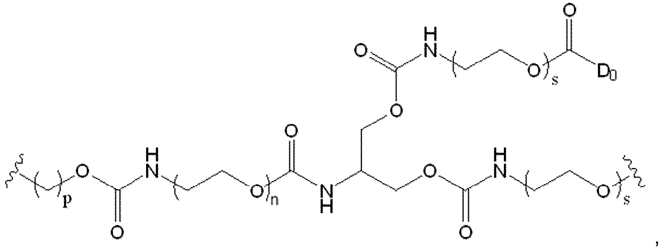 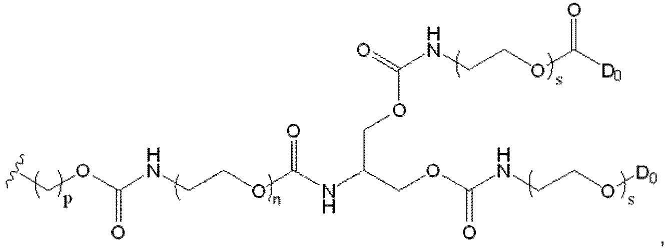 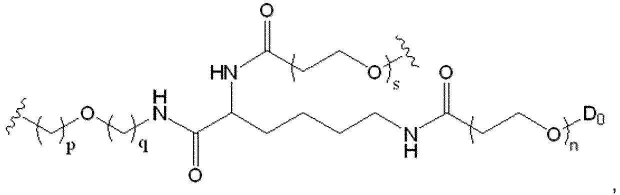 and 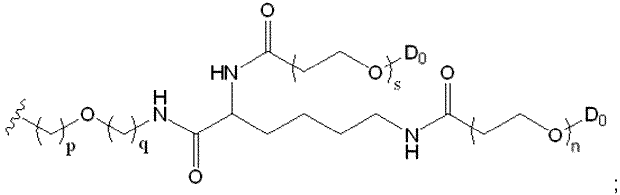 wherein p is 1, 2, or 3; q is 0, 1, or 2; r is 0 or 1; s is an integer from 0 to 37; t is 0 or 1; u is 0 or 1; v is 1, 2, or 3; and n is an integer from 1 to 37; or
   x and y are 0 and R₁ and R₂ together form =O and R₃ is 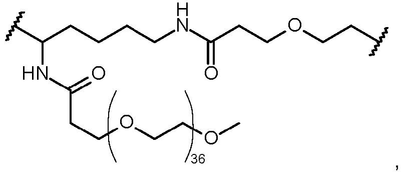
   D₀ is methyl, propargyl, or Eribulin; and
   optionally, if q, r, s, and t are 0, then x+y+z>1.

### Linker-payload moieties

The conjugate of the present invention comprises a linker-payload moiety conjugated to an antibody. The linker-payload moiety comprises a linker, L; one, two or three bridging groups, R₁, R₂, and R₃; and a payload represented by D₁, D₂ and D₃.

There may be from 1 to 6 payloads on a linker-payload moiety. Preferably, there are from one to three payloads. More preferably, there are one or two payloads, and most preferably, there is one payload on a linker-payload moiety.

In some embodiments, the linker-payload moiety has the structure:

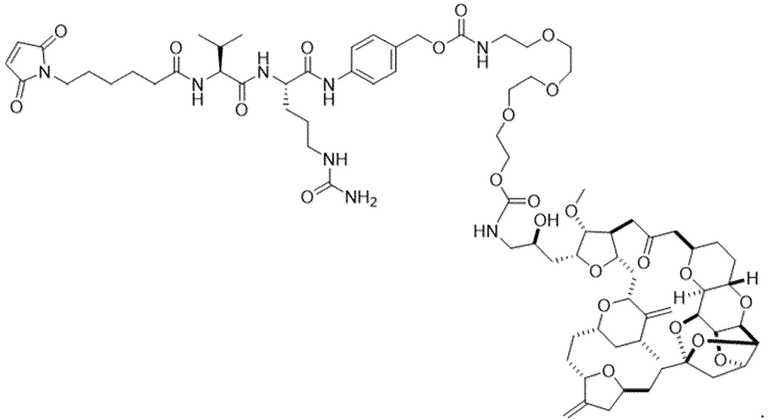

In some embodiments, the linker-payload moiety has the structure:

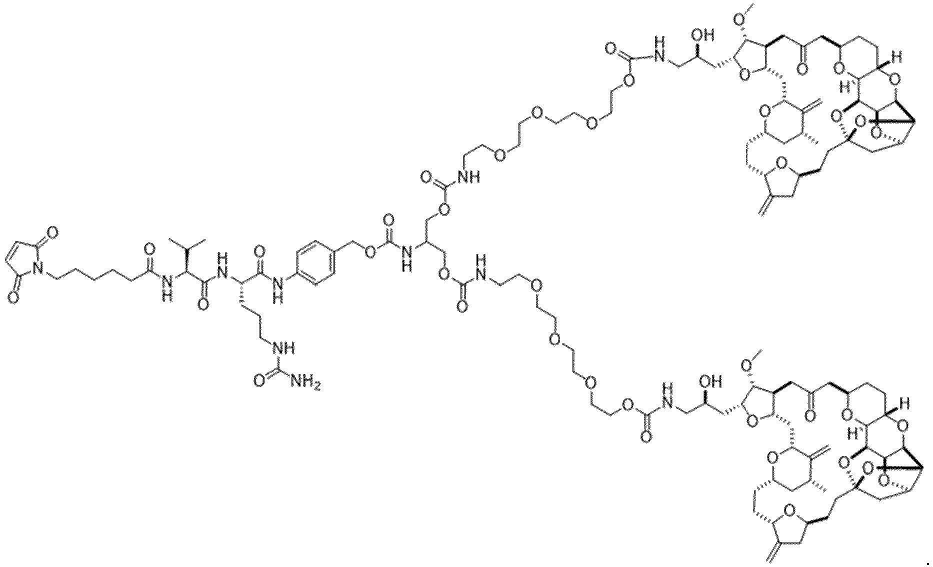

In some embodiments, the linker-payload moiety has the structure: 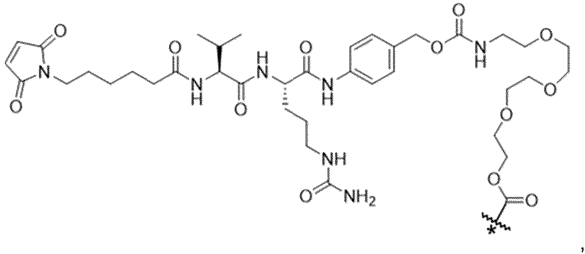 where the asterisk (*) denotes connection to an Eribulin molecule.

In some embodiments, the linker-payload moiety has the structure: 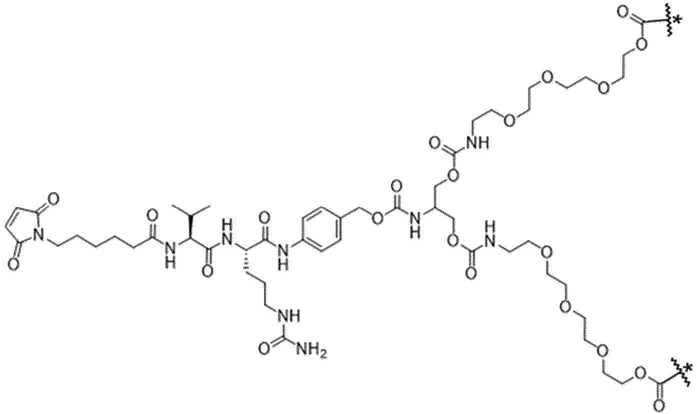 , where each asterisk (*) independently denotes connection to an Eribulin molecule.

In some embodiments, the linker is coupled to the antibody via cysteine-based conjugation. In some embodiments, the linker comprises a thiol-reactive group. In some embodiments, the linker is coupled to the antibody via reaction between cysteine residues in the antibody and a thiol-reactive functional group in the linker. In some embodiments the linker comprises a maleimide group. In some embodiments, the linker comprises a maleimidocaproyl group. The maleimidocaproyl group or maleimide group according to the present invention may be reacted with a thiol group of a cysteine residue of the antibody according to the present invention to form a sulphur-carbon bond, thereby effecting linkage of the linker to the antibody.

### Payload

In the conjugate of the present invention, the payload is generally Eribulin. In Formula (II), D₁, D₂ and D₃ (where they are present) are each Eribulin. Eribulin is connected to a bridging group, R₁, R₂, or R₃ via the amine group.

Eribulin is commercially available and has CAS no. 253128-41-5.

The linker-payload moiety comprises at least one Eribulin payload, which may be represented by one or more of D1, D2, and D3 in Formula (I). In some embodiments, the linker-payload moiety comprises more than one Eribulin payload. In some preferred embodiments, the linker-payload moiety comprises only one Eribulin payload. In some preferred embodiments, the linker-payload moiety comprises two Eribulin payloads.

The structure of Eribulin is given below.

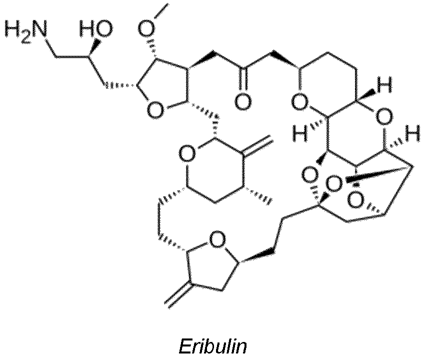

Eribulin forms a bond to the rest of the conjugate (e.g., through one or more of carbonyl, spacer (e.g. R₁), and linker (L)) via its terminal amine. That is, in the below structure, the asterisk (*) marks the point of attachment to the rest of conjugate (e.g. to R₁ (via C(O))):

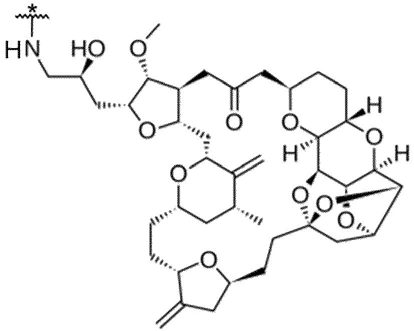

### Linker

In Formula (II), the linker, L, connects the anti-Endoglin antibody, Q, to the bridging groups R₁, R₂ and R₃.

The linker is conjugated to an amino acid residue on the antibody.

The linker may be selected from the group consisting of: 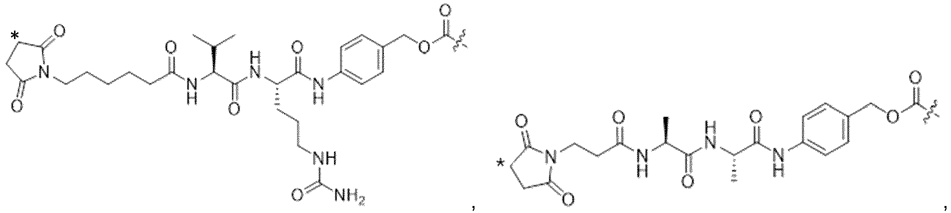 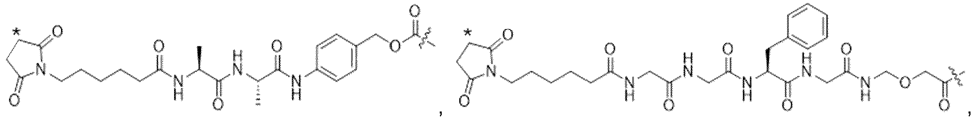 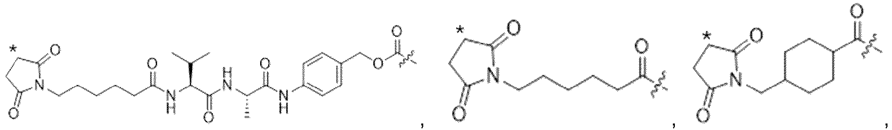 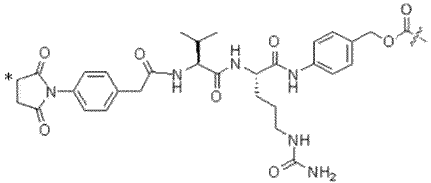 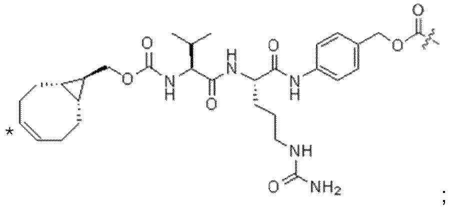 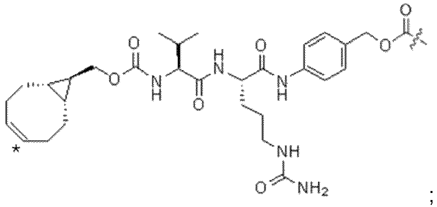 and 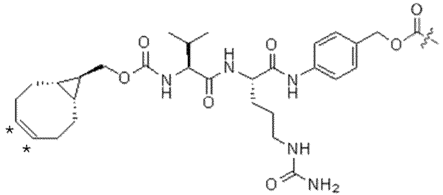 wherein * marks the point of attachment to the antibody and 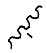 marks the point of attachment to the NH- group.

Where the point of attachment is depicted as being at the 3-position on the maleimidyl group, the point of attachment may equally be at the 4-position. That is, the below groups are considered equivalent: 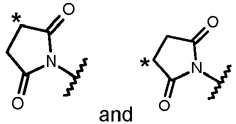 where the asterisk (*) marks the point of attachment to the antibody Q.

The linker is preferably selected from the group consisting of:

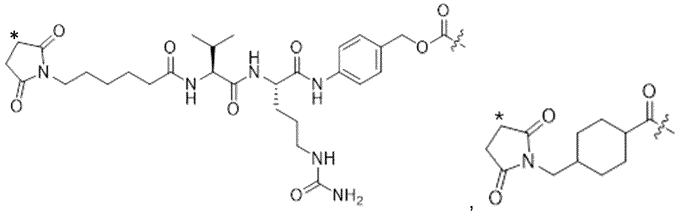

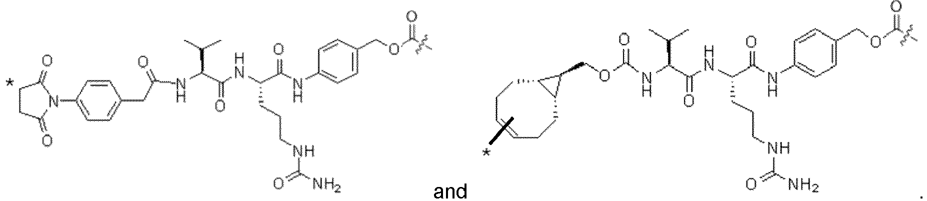

The linker is most preferably

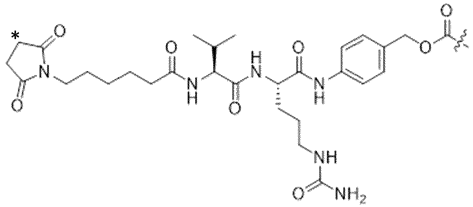

In some embodiments, L comprises the structure:

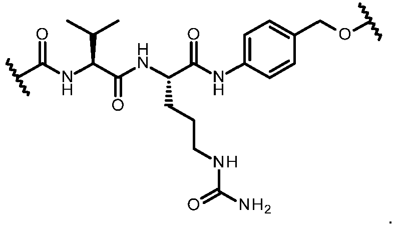

In some embodiments, L comprises the structure: 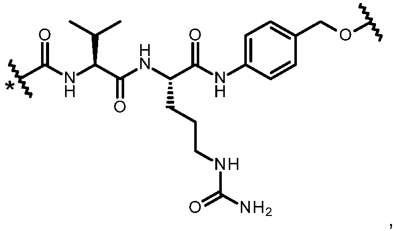
wherein a conjugating group is connected (optionally via a joining group) at the asterisk (*). The conjugating group is a group which conjugates the linker-payload to the antibody; optionally the conjugating group is a maleimide group; further optionally the joining group is an alkylene group (e.g., - (CH₂)₅-).
*X, Y and* Z
x, y and z are each independently 0 or 1, with the proviso that at least one of x, y and z is 1;

Preferably, x+y+z = 1 or 2.

More preferably, x+y+z = 1.

### Bridging group

The bridging groups, R₁, R₂ and R₃, are connected to the linker, L. The payload, Eribulin, is connected to L via a bridging group.

Each of R₁, R₂, and R₃ is independently a bond, hydrogen, 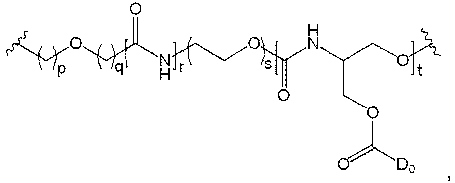 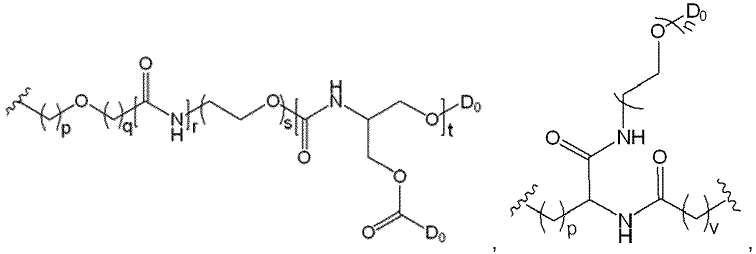 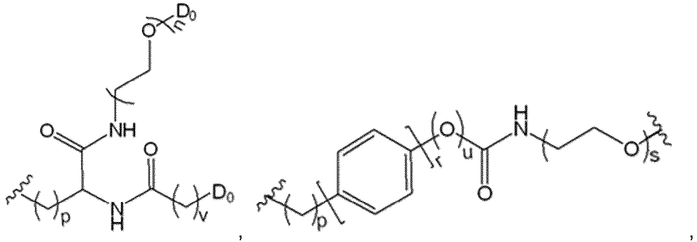 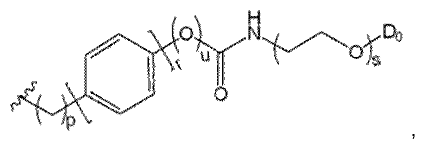 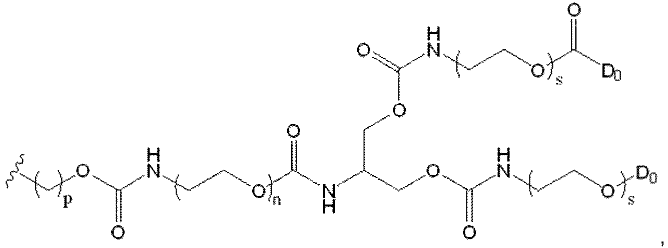 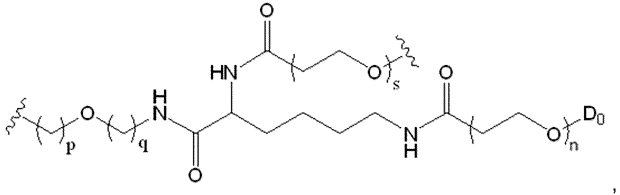 or 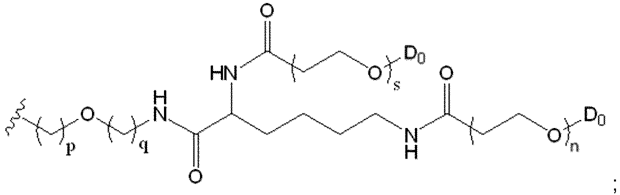 wherein p is 1, 2, or 3; q is 0, 1, or 2; r is 0 or 1; s is an integer from 0 to 37; t is 0 or 1; u is 0 or 1; v is 1, 2, or 3; and n is an integer from 1 to 37; or
x and y are 0 and R₁ and R₂ together form =O and R₃ is 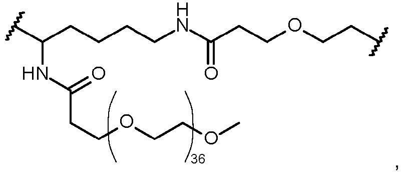
D₀ is methyl, propargyl, or Eribulin;
with a provision that at least one of R₁, R₂, and R₃ is not hydrogen;
if q, r, s, and t are 0, then x+y+z>1;
if x is 0, then R₁ is selected from: hydrogen, 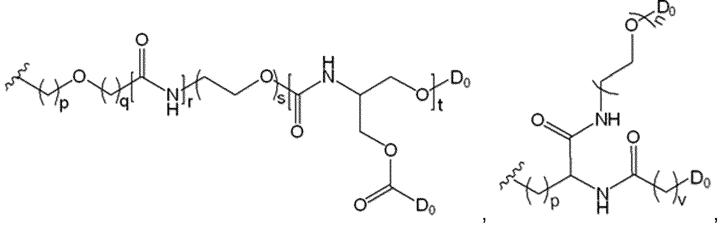 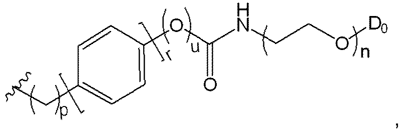 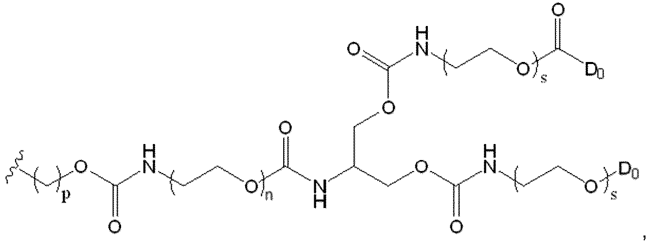 and 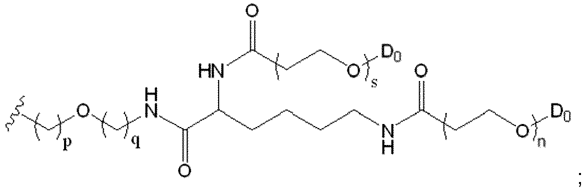
if y is 0, then R₂ is selected from: hydrogen, 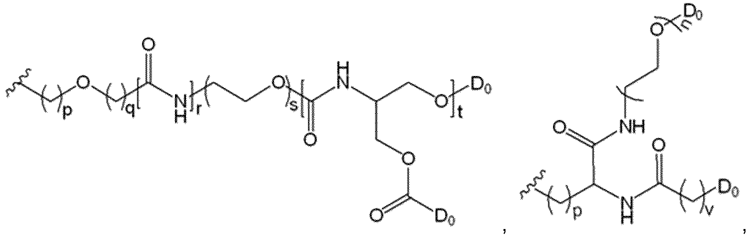 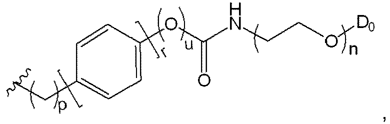 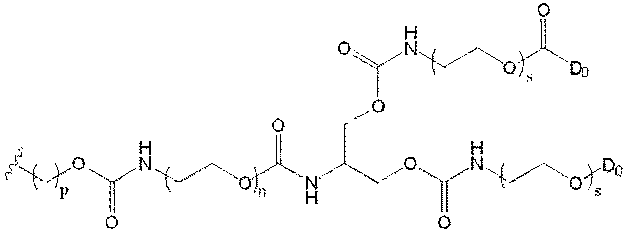 , and 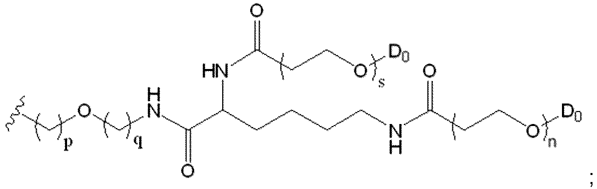
if z is 0, then R₃ is hydrogen, 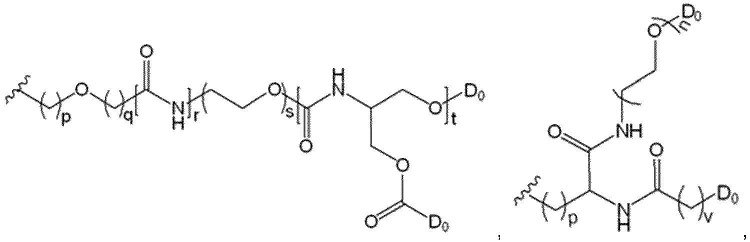 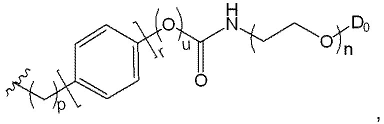 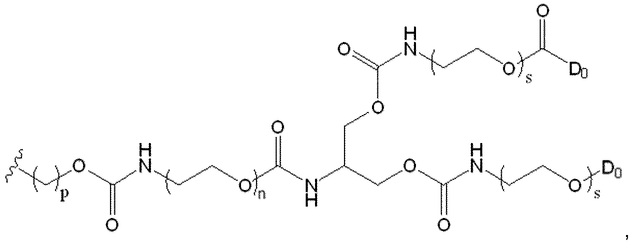 and 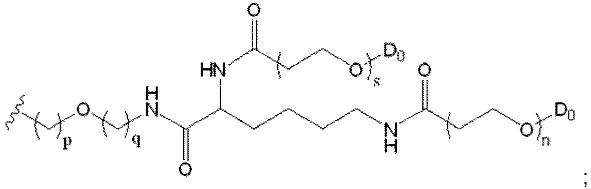
optionally, if x+y+z=1 and each of R₁, R₂, and R₃ independently is 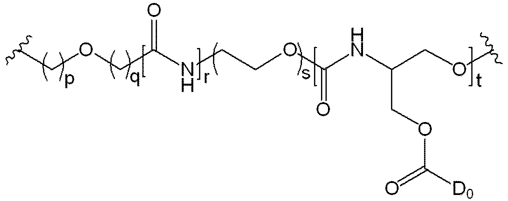 or 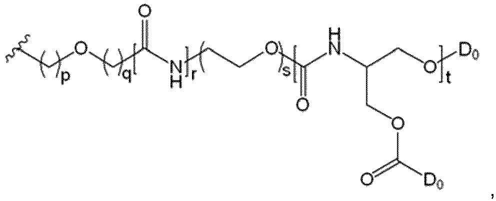 then p is not 1; and
optionally, if r, s, and t are 0 and each of R₁, R₂, and R₃ independently is 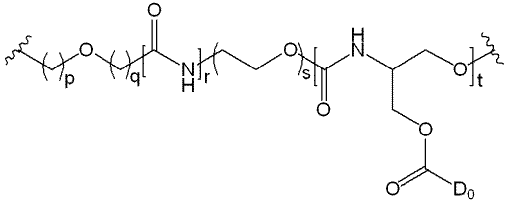 or 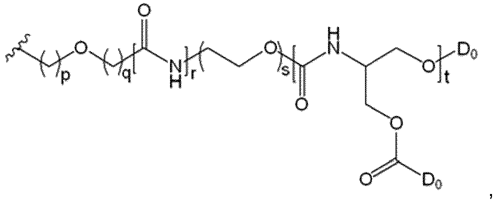 then x+y+z>1.

Wherein the point of attachment, 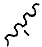 , on the far left of each group indicates the point of attachment to L.

Optionally, if x+y+z=1 and each of R₁, R₂, and R₃ independently is 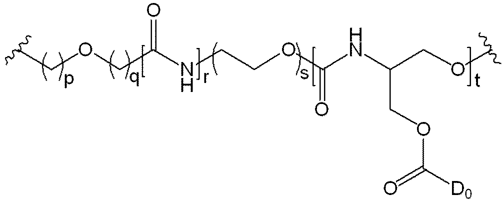 or 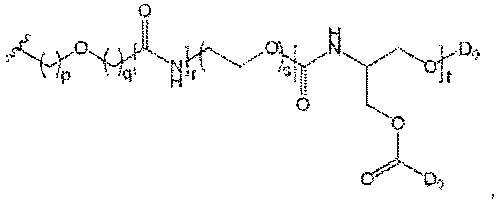 then p is not 1.

Optionally, if only one of x, y and z is 1, and the respective R₁, R₂ or R₃ is 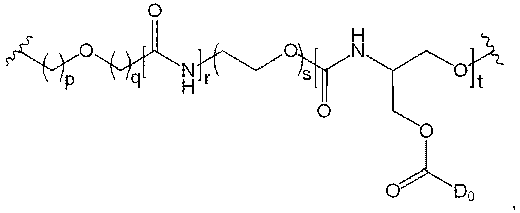 and if the remaining two of x, y, and z are 0 and the respective R₁, R₂ or R₃ is 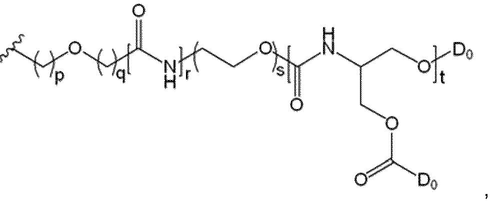 then p is not 1.

Optionally, if r, s, and t are 0 and each of R₁, R₂, and R₃ independently is 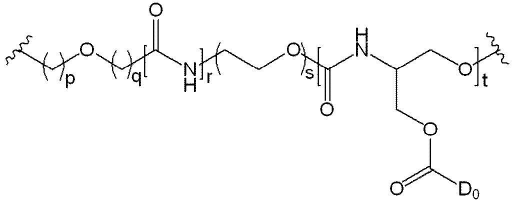 or 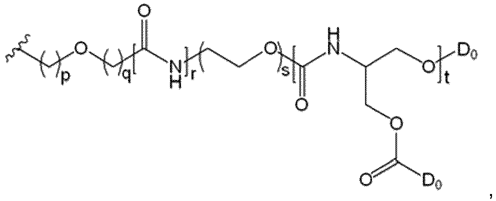 then x+y+z>1.

Preferably, if x is 0, then R₁ is hydrogen; if y is 0, then R₂ is hydrogen; and if z is 0, then R₃ is hydrogen.

Preferably, each of R₁, R₂, and R₃ is independently a bond, hydrogen, 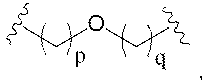 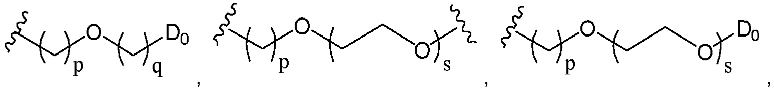 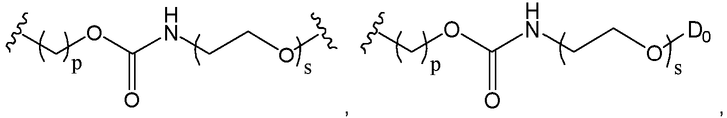 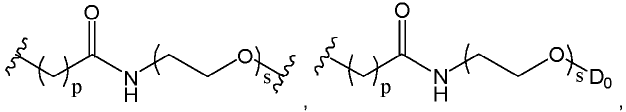 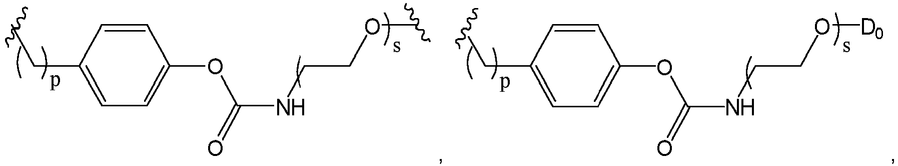 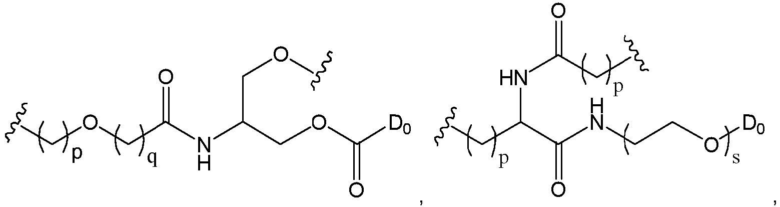 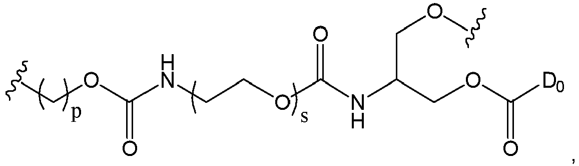 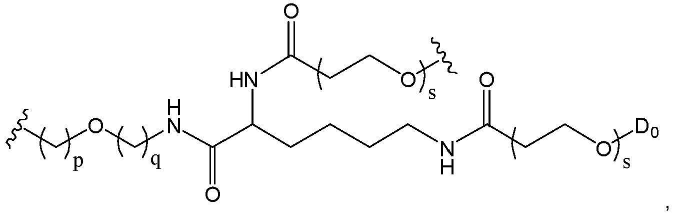 or 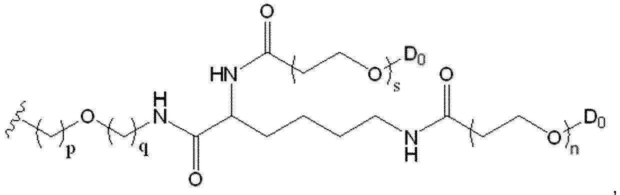 where D₀ is methyl, propargyl, or Eribulin; p is 1, 2, or 3; q is 0, 1, or 2; r is 0 or 1; s is an integer from 0 to 37; t is 0 or 1; u is 0 or 1; v is 1, 2, or 3; and n is an integer from 1 to 37.

More preferably, each of R₁, R₂, and R₃ is independently hydrogen, 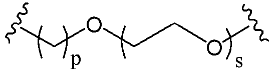 or 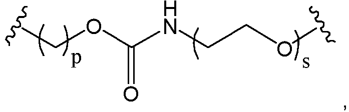 preferably wherein p is 1 and s is an integer of from 1 to 4.

Most preferably, at least one of R₁, R₂, and R₃ is 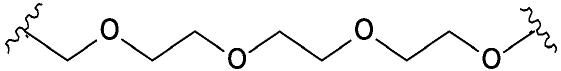 or

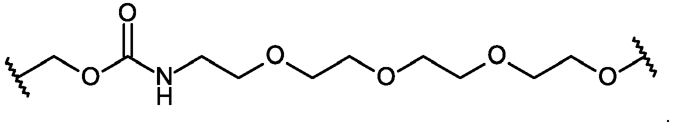

Most preferably, each of R₁, R₂, and R₃ is independently hydrogen, 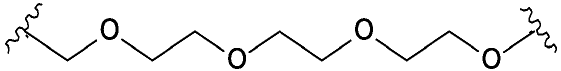 or

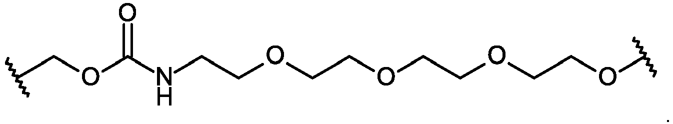

**In** one embodiment, each of R₁, R₂, and R₃ is independently hydrogen or

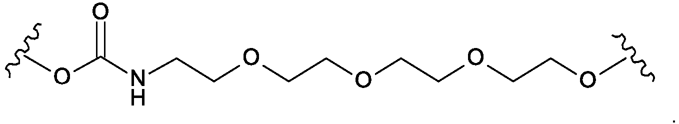

**In** one embodiment, R₁ and R₂ are hydrogen and R₃ is

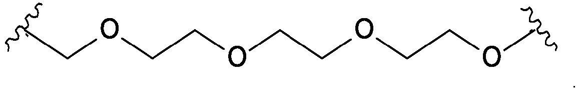

**In** another embodiment, R₁ is hydrogen and R₂ and R₃ are each

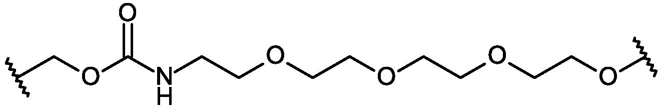

**In** another embodiment, R₁ is hydrogen and R₂ and R₃ are each

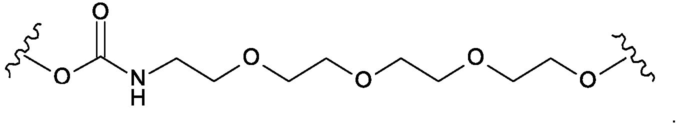

### DAR

The drug-antibody ratio (DAR) is the number of payloads (Eribulin) on each ADC. The DAR will depend on the number of payloads per linker-payload moieties conjugated, and on the number of linker-payload moieties conjugated per antibody. The DAR may also be dependent on the conditions for conjugation of the linker-payload moiety to the antibody.

In the present invention, the DAR may be from about 3 to 14, preferably from about 3 to 13, preferably from about 3 to 10, and most preferably from about 7 to 9. In some embodiments, the DAR is from about 3 to 14, from about 3 to 10, from about 3 to 6, from about 3 to 5, from about 3 to 4, from about 4 to 5, from about 5 to 10, from about 6 to 10, form about 7 to 9, from about 7 to 8, from about 8 to 9, from about 8 to 14, from about 9 to 14, from about 10 to 14, from about 11 to 14, from about 12 to 14, or from about 12 to 13.

In some embodiments, the DAR may be about 14 or less, about 13 or less, about 12 or less, about 11 or less, about 10 or less, about 9 or less, about 8 or less, about 7 or less, about 6 or less, about 5 or less, about 4 or less. In some embodiments, the DAR may be about 9 or less. In some embodiments, the DAR may be about 8 or less. In some embodiments, the DAR may be about 5 or less. In some embodiments, the DAR may be about 4 or less.

In some embodiments, the DAR may be about 4 or above, about 5 or above, about 6 or above, about 7 or above, about 8 or above, about 9 or above, about 10 or above, about 11 or above, about 12 or above, about 13 or above, about 14 or above. In some embodiments, the DAR may be about 3 or above. In some embodiments, the DAR may be about 4 or above. In some embodiments, the DAR may be about 7 or above. In some embodiments, the DAR may be about 8 or above.

In one embodiment, the DAR is from about 3 to 5.

In one embodiment, the DAR is from about 7 to 9.

In one embodiment, the DAR is from about 10 to 14.

DAR may be determined by HIC-HPLC or Reduced RPLC, for example HIC-HLPC.

### Preferred embodiments

In a preferred embodiment, the linker-payload moiety is: 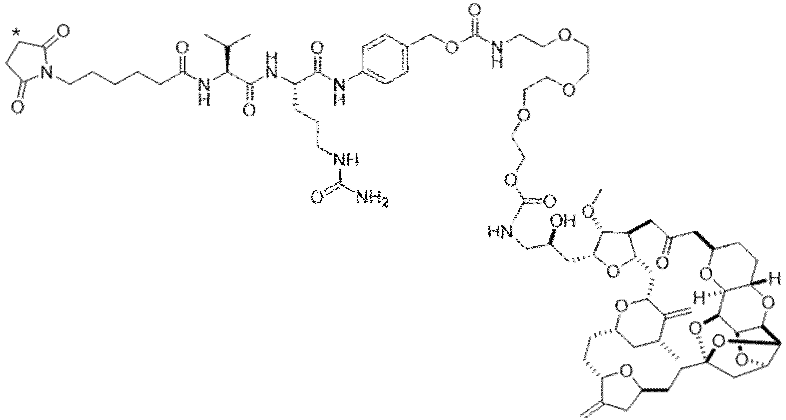 wherein * indicates the point of attachment to the antibody, Q.

In this embodiment, the DAR may be from about 3 to 5 or from about 7 to 9.

Preferably, the DAR is from about 7 to 9.

In another preferred embodiment, the linker-payload moiety is: 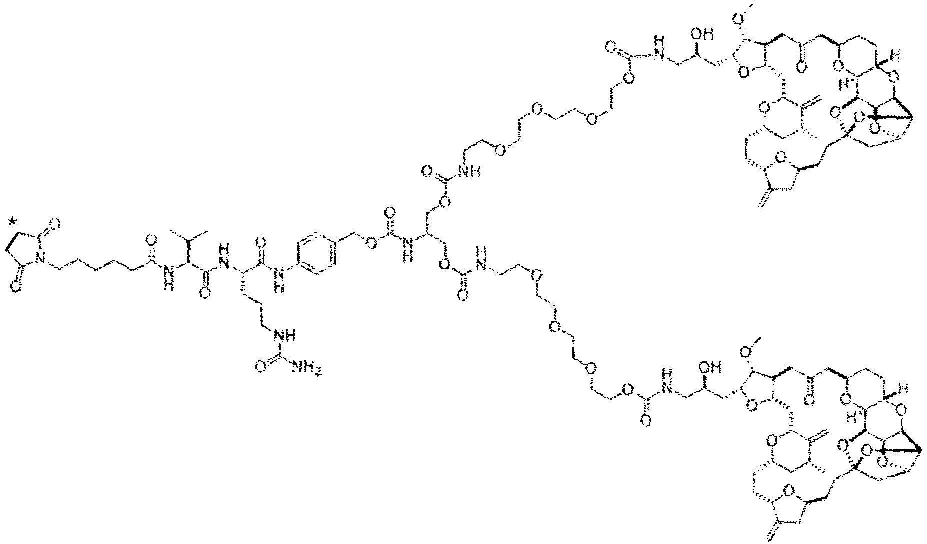 wherein * indicates the point of attachment to the antibody, Q.

In this embodiment, the DAR is preferably from about 10 to 14.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

Structures of some specific conjugates which are referred to herein are shown in Table 1. OMTX603-2, OMTX603-3, and OMTX603-4 are according to the present invention. OMTX603-2 and OMTX603-3 use the same linker-payload moiety, but differ in DAR (i.e., the number of linker-payload moieties per antibody). OMTX103 and OMTX703 are included as comparative examples.

**Table 1. Conjugates referred to herein**

| **Name** | **DAR** | **Structure** |
|---|---|---|
| OMTX603-2 | 4.15 | 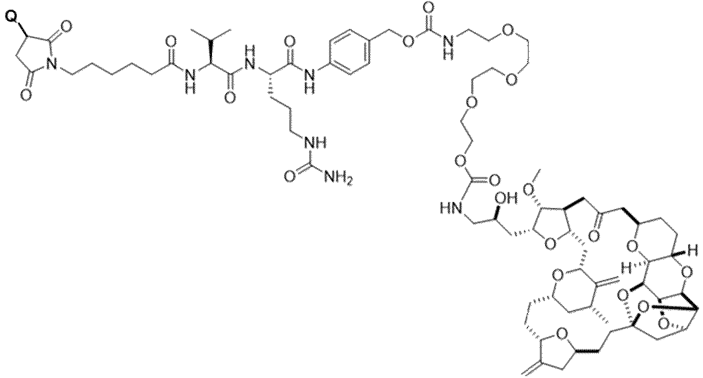 |
| OMTX603-3 | 7.97 | 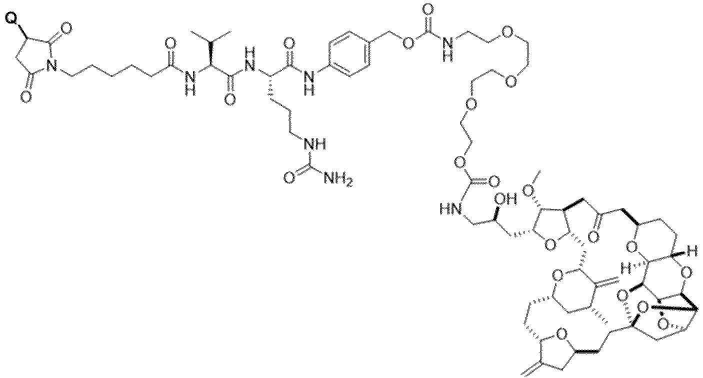 |
| OMTX603-4 | 12.76 | 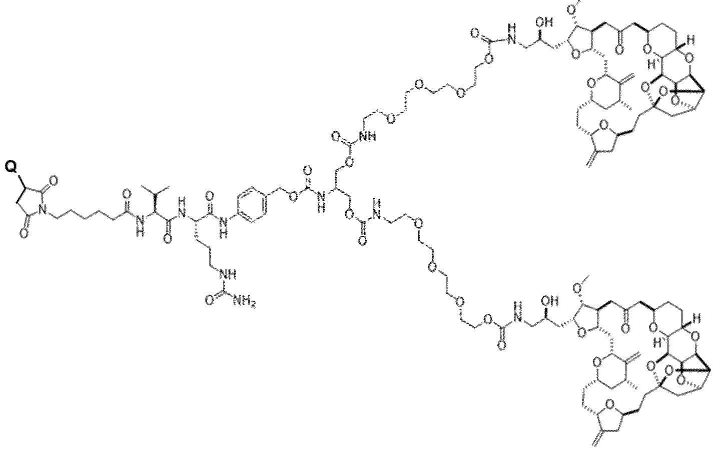 |
| OMTX103 (comparative) | 3.75 | 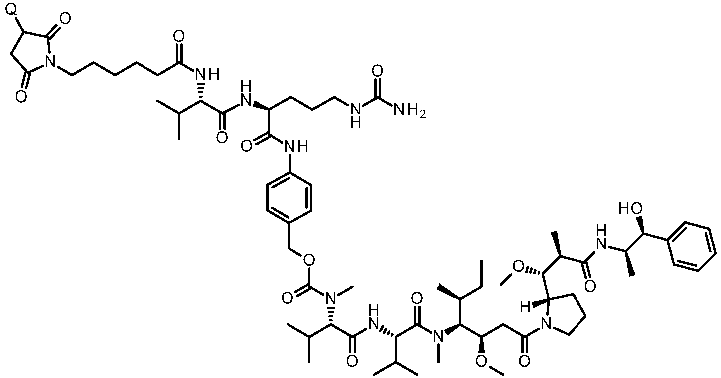 |
| OMTX703 (comparative) | 3.8 | 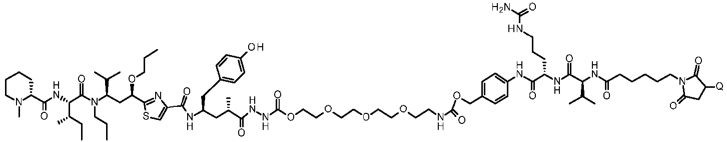 |

As set out in Table 1, OMTX603-2 has a DAR of about 4.15, OMTX603-3 has a DAR of about 7.97, and OMTX603-4 has a DAR of about 12.76.

### EXAMPLE 1: Production of anti-ENG ADCs

Three ADCs were generated, each ADC comprising an anti-endoglin antibody (OMTX003) and Eribulin as a payload; OMTX603-2, OMTX603-3 and OMTX603-4.

OMTX003 was specific for the extracellular region of human ENG (amino acids 26-561 of SEQ ID NO: 25) and may otherwise be referred to as A5-IgG1. The amino acid sequences of anti-human ENG IgG1 A5 (A5-IgG1) heavy chain (HC) and light chain (LC), respectively are shown below:

### Anti-human Endoglin A5-IgG1-HC:

METDTLLLWVLLLWVPGSTG
EVQLLESGGGLVQPGGSLRLSCAASGFTFSSYAMSWVRQAPGKGLEWVSAIYGSDGDTTYADSVKGRF
TISRDNSKNTLYLQMNSLRAEDTAVYYCARVFYTAGFDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPPVAGPSVFLFPPKPKDTLMISRTPEVTCVVVDVSHEDPEVKFN
WYVDGVEVHNAKTKPREEQYNSTYRVVSVLTVLHQDWLNGKEYKCKVSNKGLPSSIEKTISKAKGQPRE
PQVYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO: 1)

| | |
|---|---|
| aa | 447 |
| *MW of processed HC* | *48,703* |
| *Theoretical pl* | *8.36* |
| *Potential glycosylation site (double underlined):* | *N297* |

*Mutations leading to ADCC and CDC deficiency are shown in bold italics (see also* WO 99/58572) *Signal sequence is shown boxed*
*VH domain is underlined; CDRH1-H3 are shown in bold and curved underlined.*

### Anti-human Endoglin A5-IgG1-LC:

METETDTLLLWVLLLWVPGSTG
DIELTQSPSSLSASVGDRVTITCRASQSISSSLNWYQQKPGKAPKLLIYAASSLQSGVPSRFSGSGSGTD
FTLTISSLQPEDFATYYCQQAPAKPPTFGQGTKLEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC (SEQ ID NO: 2)

| | |
|---|---|
| aa | 214 |
| *MW of processed HC* | *23,113* |
| *theoretical pl* | *7.76* |
| *signal sequence is boxed* | |

*VL domain is underlined; CDRL1-L3 are shown in bold and curved underlined.*
A5-IgG1-HC - without signal sequence: 
EVQLLESGGGLVQPGGSLRLSCAASGFTFSSYAMSWVRQAPGKGLEWVSAIYGSDGDTTYADSVKGRF
TISRDNSKNTLYLQMNSLRAEDTAVYYCARVFYTAGFDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
 
SNTKVDKKVEPKSCDKTHTCPPCPAPPVAGPSVFLFPPKPKDTLMISRTPEVTCVVVDVSHEDPEVKFN
WYVDGVEVHNAKTKPREEQYNSTYRVVSVLTVLHQDWLNGKEYKCKVSNKGLPSSIEKTISKAKGQPRE
PQVYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO: 3)
A5-lgG1-LC - without signal sequence:

DIELTQSPSSLSASVGDRVTITCRASQSISSSLNWYQQKPGKAPKLLIYAASSLQSGVPSRFSGSGSGTD
FTLTISSLQPEDFATYYCQQAPAKPPTFGQGTKLEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC (SEQ ID NO: 4)

### Preparation of Linker-Payload

A person having ordinary skill in the chemical art would be able to make a linker-payload conjugate according to the present invention, by these methods or similar methods or other methods practiced by a person skilled in the art. In general, starting components are commercially available chemicals and can be obtained from commercial sources or can be made according to organic synthesis techniques known to those skilled in this art, starting from commercially available chemicals and/or from compounds described in the chemical literature.

In general, the compounds used in the reactions described herein can be made according to organic synthesis techniques known to those skilled in this art, starting from commercially available chemicals and/or from compounds described in the chemical literature. "Commercially available chemicals" can be obtained from standard commercial sources including Acros Organics (Pittsburgh PA), Aldrich Chemical (Milwaukee WI, including Sigma Chemical and Fluka), Apin Chemicals Ltd. (Milton Park UK), Avocado Research (Lancashire U.K.), BDH Inc. (Toronto, Canada), Bionet (Cornwall, U.K.), Chemservice Inc. (West Chester PA), Crescent Chemical Co. (Hauppauge NY), Eastman Organic Chemicals, Eastman Kodak Company (Rochester NY), Fisher Scientific Co. (Pittsburgh PA), Fisons Chemicals (Leicestershire UK), Frontier Scientific (Logan UT), ICN Biomedicals, Inc. (Costa Mesa CA), Key Organics (Cornwall U.K.), Lancaster Synthesis (Windham NH), Maybridge Chemical Co. Ltd. (Cornwall U.K.), Parish Chemical Co. (Orem UT), Pfaltz & Bauer, Inc. (Waterbury CN), Polyorganix (Houston TX), Pierce Chemical Co. (Rockford IL), Riedel de Haen AG (Hanover, Germany), Spectrum Quality Product, Inc. (New Brunswick, NJ), TCI America (Portland OR), Trans World Chemicals, Inc. (Rockville MD), and Wako Chemicals USA, Inc. (Richmond VA).

Methods known to one of ordinary skill in the art can be identified through various reference books and databases. Suitable reference books and treatise that detail the synthesis of reactants useful in the preparation of compounds of the present disclosure, or provide references to articles that describe the preparation, include for example, Synthetic Organic Chemistry, John Wiley & Sons, Inc., New York; S. R. Sandler et al., Organic Functional Group Preparations, 2nd Ed., Academic Press, New York, 1983; H. O. House, Modern Synthetic Reactions, 2nd Ed., W. A. Benjamin, Inc. Menlo Park, Calif. 1972; T. L. Gilchrist, Heterocyclic Chemistry, 2nd Ed., John Wiley & Sons, New York, 1992; J. March, Advanced Organic Chemistry: Reactions, Mechanisms and Structure, 4th Ed., Wiley Interscience, New York, 1992.

General methods for the preparation of compounds as disclosed herein may be derived from reactions and the reactions may be modified by the use of appropriate reagents and conditions, for the introduction of the various moieties found in the formulae as provided herein. As a guide the following synthetic methods may be utilized.

The linker-Eribulin moieties for OMTX603-2, OMTX603-3 and OMTX603-4 were prepared as follows.

### Synthesis of linker-payload for OMTX603-2 and OMTX603-3 (Mal-Val-Cit-PAB-PEG₄-Eribulin)

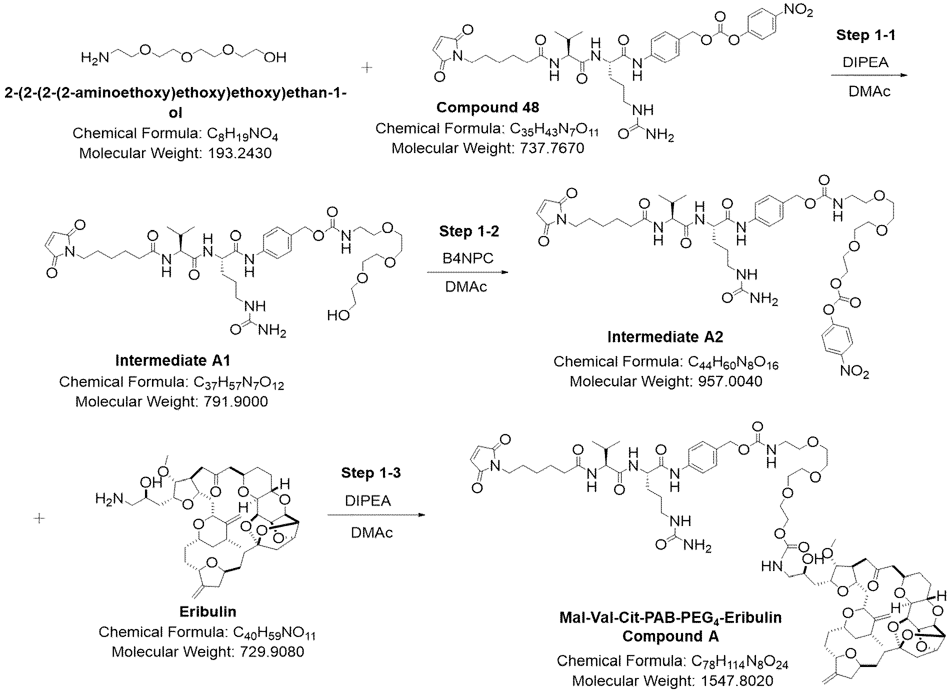

### Scheme I: Synthesis of Compound A (Mal-Val-Cit-PAB-PEG4-Eribulin)

¹H-NMR (400MHz, DMSO-*d*₆) δ 9.96 (s, 1H), 8.05 (d, *J* = 7.6 Hz, 1H), 7.78 (d, *J* = 8.8 Hz, 1H), 7.58 (d, *J* = 8.8 Hz, 2H), 7.27 (d, *J = 8.8* Hz, 2H), 7.19 (t, *J =* 4.9 Hz, 1H), 7.04 (t, *J =* 6.0 Hz, 1H), 6.99 (s, 1H), 5.96 (t, *J* = 5.4 Hz, 1H), 5.39 (s, 2H), 5.32 (t, *J* = 4.9 Hz, 1H), 5.05 (s, 1H), 5.00 (s, 1H), 4.93 (s, 2H), 4.83 (s, 1H), 4.75 (s, 1H), 4.63 (m, 1H), 4.55 (t, *J* = 4.0 Hz, 1H), 4.51 (d, *J* = 5.2 Hz, 1H), 4.38 (m, 1H), 4.33 (d, *J =* 4.0 Hz, 1H), 4.26 (m, 1H), 4.22-4.13 (m, 2H), 4.10 (s, 3H), 3.08~4.06 (m, 3H), 3.85-3.65 (m, 4H), 3.57-3.52 (m, 3H), 3.50 (s, 3H), 3.49 (s, 2H), 3.43-3.38 (m, 4H), 3.27-3.24 (m, 5H), 3.13 (m, 2H), 3.05-2.90 (m, 5H), 2.89-2.63 (m, 4H), 2.60-2.52 (m, 1H), 2.37-2.05 (m, 7H), 2.05-1.84 (m, 8H), 1.79-1.40 (m, 10H), 1.40-1.10 (m, 10H), 1.07-1.00 (m, 6H), 0.89-0.78 (m, 6H). ESI MS calculated [M+H]⁺: 1547.8, observed: 1547.7.

**Step 1-1:** Compound **48** (0.4 g, 0.54 mmol, 1.0 equiv.) was dissolved in dimethylacetamide (DMAc, 2.0 mL). 2-(2-(2-(2-aminoethoxy)ethoxy)ethoxy)ethan-1-ol (180.0 mg, 0.92 mmol, 1.7 equiv.) was also dissolved in DMAc (1.0 mL) with addition of N,N-diisopropylethylamine (DIPEA, 140.0 mg, 1.08 mmol, 2.0 equiv.). The 2-(2-(2-(2-aminoethoxy)ethoxy)ethoxy)ethan-1-ol solution was slowly added to the Compound **48** solution and stirred at 15-25°C for 16 hours. After the completion of the reaction was confirmed by ultra-performance liquid chromatography (UPLC), DMAc was removed by vacuum. The residue was further purified by hydrophilic-interaction chromatography (HILIC) so as to give Intermediate A1 with a yield of 69%.

**Step 1-2:** Intermediate A1 (180.0 mg, 0.22 mmol, 1.0 equiv.) was dissolved in DMAc (2.7 mL) and DIPEA (120.0 mg, 0.898 mmol, 4.0 equiv.) was added thereto. Bis(4-nitrophenyl) carbonate (B4NPC, 240.0 mg, 0.78 mmol, 3.5 equiv.) was added to the solution and stirred at 15-25°C for 2.5 hours. After the completion of the reaction was confirmed by UPLC, the solution was slowly added to ethyl acetate at 0-10°C under nitrogen atmosphere and stirred at the same temperature for 15 minutes, followed by 10 minutes at 15-25°C to precipitate crude Intermediate A2. The crude was collected, rinsed with ethyl acetate repeatedly, filtered, and vacuum-dried to give Intermediate A2 with a yield of 70%.

**Step 1-3:** Eribulin (40.0 mg, 0.05 mmol, 1.05 equiv.) was dissolved in DMAc (0.5 mL) and DIPEA (10.0 mg, 0.08 mmol, 1.5 equiv.) was added thereto. The solution was cooled down to 0-10°C and Intermediate A2 (50.0 mg, 0.05 mmol, 1.0 equiv.) was added thereto. The solution was stirred at 15-25°C for 4.5 hours. After the completion of the reaction was confirmed by UPLC, the solution was purified by HILIC to afford Compound A with a yield of 70%.

Compound 48 is commercially available from vendors such as Sigma Aldrich.

### Synthesis of OMTX603-4 linker-payload moiety (Mal-Val-Cit-PAB-(PEG₄-Eribulin)₂)

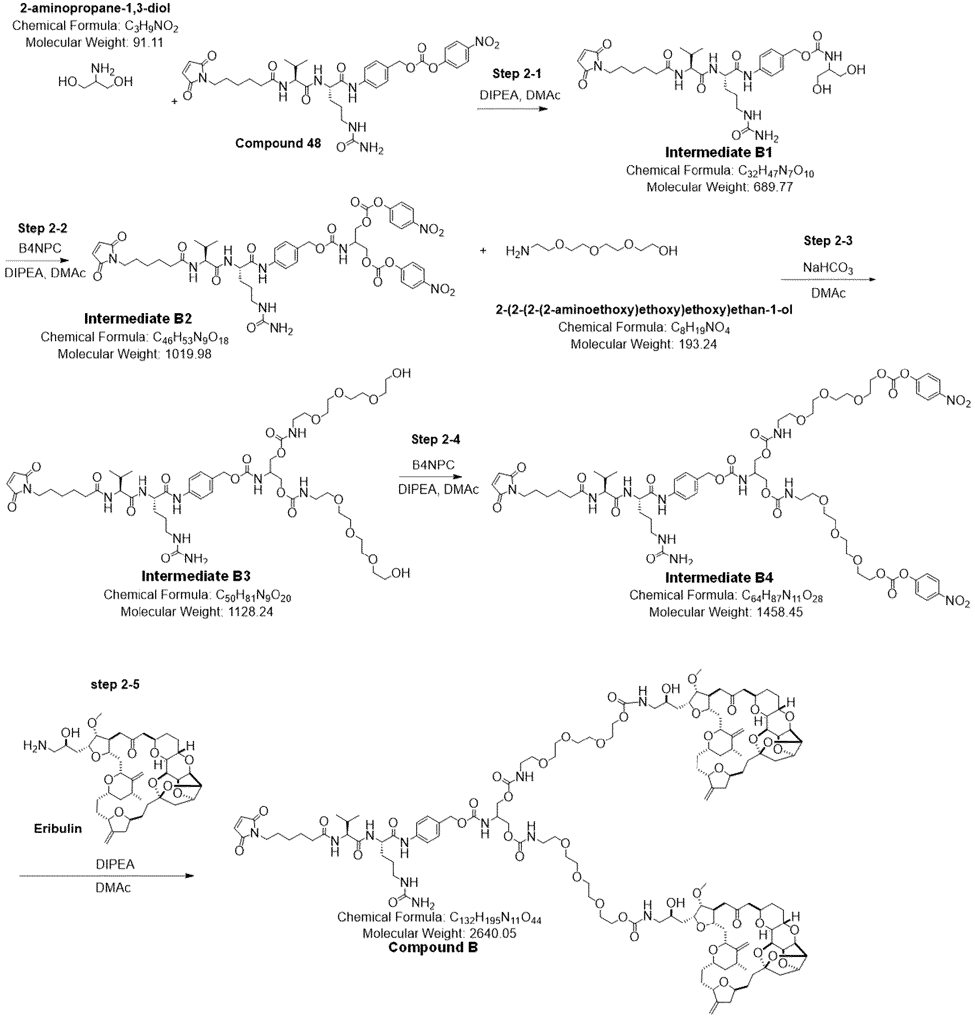

### Scheme II: Synthesis of Compound B (Mal-Val-Cit-PAB-(PEG4-Eribulin)₂)

¹H-NMR (400MHz, DMSO-*d*₆) δ 9.96 (s, 1H), 8.04 (d, *J* = 7.6 Hz, 1H), 7.77 (d, *J* = 8.8 Hz, 1H), 7.59 (d, *J* = 8.4 Hz, 2H), 7.28 (d, *J* = 8.4 Hz, 2H), 7.16-7.08 (m, 2H), 7.02 (t, *J* = 5.6 Hz, 2H), 6.99 (s, 2H), 5.95 (t, *J* = 5.2 Hz , 1H), 5.38 (s, 2H), 5.08-5.02 (m, 2H), 5.02-4.97 (m, 2H), 4.97-4.92 (m, 2H), 4.86-4.80 (m, 2H), 4.78-4.72 (m, 2H), 4.66-4.60 (m, 2H), 4.55 (t, *J* = 4.0 Hz, 2H), 4.53-4.46 (m, 2H), 4.38 (m, 1H), 4.34-4.22 (m, 3H), 4.22-4.13 (m, 4H), 4.13-3.94 (m, 4H), 3.94-3.64 (m, 9H), 3.59-3.45 (m, 18H), 3.42-3.34 (m, 6H), 3.28-3.23 (m, 10H), 3.15-3.06 (m, 5H), 3.06-2.88 (m, 6H), 2.87-2.52 (m, 10H), 2.37-1.84 (m, 32H), 1.78-1.12 (m, 43H), 1.04 (d, *J* = 6 Hz, 6H), 0.89-0.78 (m, 6H). ESI MS calculated [M+2H]²⁺: 1320.2, observed: 1320.9.

**Step 2-1:** Compound **48** (200.0 mg, 0.27 mmol, 1.0 equiv.) was dissolved in DMAc (1.2 mL). 2-aminopropane-1,3-diol (25.0 mg, 0.27 mmol, 1.0 equiv.) was also dissolved in DMAc (0.8 mL) with addition of DIPEA (32.0 mg, 0.27 mmol, 1.0 equiv.). The 2-aminopropane-1,3-diol solution was slowly added to the Compound **48** solution and stirred at 15-25°C for 3 hours. After the completion of the reaction was confirmed by UPLC, the solution was slowly added to ethyl acetate at 15-25°C and stirred for 1 hour to precipitate crude Intermediate B1. The crude was collected, rinsed with ethyl acetate repeatedly, and purified by HILIC so as to give Intermediate B1 with a yield of 60%.

**Step 2-2:** Intermediate B1 (0.4 g, 0.58 mmol, 1.0 equiv.) and B4NPC (Bis(4-nitrophenyl) carbonate, 1.1 g, 3.50 mmol, 6.0 equiv.) were dissolved in DMAc (4.0 mL). The mixture was cooled down to 0-10°C and DIPEA (140.0 mg, 1.08 mmol, 2.0 equiv.) was added thereto. The reaction was performed at 15-25°C. After the completion of the reaction was confirmed by UPLC, n-heptane/ethyl acetate (1:1 v/v) solution was added in the mixture to precipitate crude Intermediate B2. The crude was collected, rinsed with n-heptane/ethyl acetate (1:1 v/v) solution, filtered, and vacuum-dried to give Intermediate B2 with a yield of 91%.

**Step 2-3:** Intermediate B2 (200.0 mg, 0.20 mmol, 1.0 equiv.) was dissolved in DMAc (2.0 mL), and NaHCO₃ (33.0 mg, 0.39 mmol, 2.0 equiv.) was added thereto and the mixture was cooled down to 0-10°C. 2-(2-(2-(2-aminoethoxy)ethoxy)ethoxy)ethan-1-ol (103.0 mg, 0.53 mmol, 2.7 equiv.) was dissolved in DMAc (2.7 mL) and was slowly added to the Intermediate B2 solution. The mixture was stirred at 15-25°C for 2 hours. After the completion of the reaction was confirmed by UPLC, n-heptane/ethyl acetate (1:1 v/v) solution was added in the mixture to precipitate crude Intermediate B3. The crude was collected, rinsed with n-heptane/ethyl acetate (1:1 v/v) solution repeatedly, and purified by HILIC so as to give Intermediate B3 with a yield of 36%.

**Step 2-4:** Intermediate B3 (49.0 mg, 0.04 mmol, 1.0 equiv.) and B4NPC (106.0 mg, 0.35 mmol, 8.0 equiv.) were dissolved in DMAc (0.7 mL). The mixture was cooled down to 0-10°C and DIPEA (45.0 mg, 0.35 mmol, 8.0 equiv.) was added thereto. The mixture was stirred at 15-25°C for 2 hours. After the completion of the reaction was confirmed by UPLC, n-heptane/ethyl acetate (1:1 v/v) solution was added in the mixture to precipitate crude Intermediate B4. The crude was collected, rinsed with n-heptane/ethyl acetate (1:1 v/v) solution repeatedly, filtered and vacuum-dried to give Intermediate B4 with a yield of 71%.

**Step 2-5:** Intermediate B4 (49.0 mg, 0.03 mmol, 1.0 equiv.) was dissolved in DMAc (2.0 mL). Eribulin (57.0 mg, 0.08 mmol, 2.30 equiv.) was dissolved in DMAc (0.5 mL) and DIPEA (14.0 mg, 0.11 mmol, 3.3 equiv.) was added thereto. The Eribulin solution was slowly added to the Intermediate B4 solution and stirred at 15-25°C for 1 hour. After the completion of the reaction was confirmed by UPLC, ethyl acetate was added in the mixture to precipitate crude Compound B. The crude was collected, rinsed with ethyl acetate repeatedly, and purified by HILIC so as to give Compound B with a yield of 25%.

### Conjugation

The payload-linker moieties were conjugated to the anti-Endoglin antibody (OMTX003) to form the ADCs as follows:

### OMTX603-2

The anti-Endoglin antibody, which has been placed in a solution (i.e. conjugation buffer) of 10-50 mM NaPi or sodium phosphate or histidine or sodium succinate, 100-200 mM NaCl, 100-200 mM KCI, 2-20 mM EDTA, pH 7.0-7.5, was taken into a glass reaction bottle. 1.8-3.6 eq TCEP was added thereto to conduct the reduction reaction. The reduction reaction was conducted in the reaction solution at 18-25 °C for 1.5-3.5 hours, and the estimated DAR was calculated by hydrophobic interaction chromatography (HIC)-HPLC. After the reduction reaction was complete, 5-15% DMSO or DMA or ACN and 4.0-6.0 eq Compound A were added. After 0.5-2 hours of reaction, a sample was taken to confirm the DAR value. Afterwards, a buffer solution including 10-50 mM succinic acid or sodium citrate or sodium acetate or/and arginine, pH 4.5-6.0, was added to dilute the reaction solution 3-6 times and to terminate the conjugation reaction.

The diluted OMTX603-2 ADC mixture was filtered by 0.2 µm filter, followed by diafiltration concentration and purification to a buffer solution including 10-50 mM succinic acid or sodium citrate or sodium acetate or/and arginine, pH 4.5-6.0, with 30-100 kDa filter membrane to remove Compound A, impurities related to Compound A and residual solvent, e.g., DMSO, DMA or ACN. After the concentration was adjusted to 10-30 mg/mL, excipient (50-65% sucrose, and 0.02-0.4% tween 20 or tween 80) was added and the mixture was filtered by 0.2 µm filter to give OMTX603-2 ADC DS. Finally, a sample was taken to confirm the concentration, the DAR value (HIC-HPLC or Reduced RPLC) and HMW (%) (High Molecular Weight species, measured using SEC-HPLC). The product was stored under -20 to -80°C with a yield of 93-96%.

The average DAR of the representative batch of OMTX603-2 ADC was 4.15 (measured using HIC-HPLC), with purity of 94.74%.

### OMTX603-3

OMTX603-3 was synthesized in the same way as OMTX603-2, except that :
"1.8-3.6 eq TCEP", "4.0-6.0 eq Compound A" and "OMTX603-2 ADC" were replaced by:
"5.5-9.0 eq TCEP", "8.0-13.0 eq Compound A" and "OMTX603-3 ADC",
   respectively. The product was obtained with a yield of approximately 97%. The average DAR of the representative batch of OMTX603-3 ADC was 7.97 with purity of 94.47%.

### OMTX603-4

OMTX603-4 was synthesized in the same way as OMTX603-2, except that:
"4.0-6.0 eq Compound A" and "OMTX603-2 ADC"
   were replaced by:
"4.0-6.0 eq Compound B" and "OMTX603-4 ADC",
   respectively. The product was obtained with a yield of 91-98%. The average DAR of the representative batch of OMTX603-4 ADC was 12.76, with purity of 86.57%.

### EXAMPLE 2: In vitro characterization of the ADCs; binding and cytotoxicity

Cell binding of OMTX 603-2, 603-3 and 603-4 was analyzed via flow cytometry using endoglin-expressing HT1080-WT cells. HT-1080 human cells are epithelial cells derived from connective tissue from a patient with Fibrosarcoma that can be used in research and assay development. Relative mean fluorescence intensity (MFI) was measured to assess binding. The three OMTX603 ADCs were evaluated in comparison with OMTX703, an ADC comprising an endoglin-targeted antibody conjugated to a cytolysin payload. OMTX703 (see Table 1) is disclosed in WO 2015/118031 and also comprises OMTX003, but with a different linker and payload to the ADC of the present invention.

The relative MFI is calculated as follows: $relative MFI = \frac{\left({value}_{sample}-\left({value}_{detection antibody}-{value}_{cells}\right)\right)}{{value}_{cells}}$ Where: valueₛₐₘₚₗₑ is obtained by testing the titration of the antibodies; value_{detection antibody} is obtained by testing the cells only in the presence of the detection antibody; and value_{cells} is obtained by testing the cells on their own (without adding any agent).

MFI values were obtained by flow cytometry analysis using a MACSQuant^{®} analyzer from Miltenyi. For the detection of cell-bound antibodies (MFI_{construct}), one detection antibody (anti-human-Fc-PE for the detection of human IgG molecules) was used in the flow cytometry analysis. As a control, the detection antibody was also tested with cells (MFI_{detection control}), and the MFI was also calculated for cells without adding any antibody (MFI_{cells}).

OMTX703 and OMTX603-2/3/4 showed concentration-dependent binding to HT1080-WT cells in the subnanomolar range, with EC50 values of 123 pM for OMTX703, 566 pM for OMTX603-2, 156 pM for OMTX603-3 and 79 pM for OMTX603-4 (Table 2). OMTX603-2 and OMTX603-3 showed higher florescence intensity, followed by OMTX703 and OMTX603-4 (Figure 1A).

Next, the cytotoxic activity of the different ADCs (OMTX703 and OMTX603-2/3/4) was analyzed using a cell viability assay performed upon HT1080-WT cells. Results are shown in Figure 1B.

All four ADCs showed a concentration-dependent killing of HT1080-WT cells following incubation for 3 days (Figure 1B). The respective IC50 values after the three-day incubation were: 649,900 ± 616,739 pM for OMTX703, 23 ± 0.4 pM for OMTX603-2 and 6 ± 0.04 pM for OMTX603-3 and 5 ± 0.05 pM for OMTX603-4 (Table 2). OMTX603-4 and OMTX603-3 showed the greatest cytotoxicity compared to OMTX603-2 and OMTX703 ADC.

**Table 2: Overview of all ADC molecules concerning binding using flow cytometry (FC) and induction of cytotoxicity using cell viability assay (incubated for 72h).**

| Values are mean ± SD: n=1 or 2. | | |
|---|---|---|
| | **EC50 (FC - HT1080-WT) [pM]** | **IC50 (Cytotox - HT1080-WT) [pM]** |
| **OMTX703** | 123 | 649,900 ± 616,739 |
| **OMTX603-2** | 566 | 23 ± 0.4 |
| **OMTX603-3** | 156 | 6 ± 0.04 |
| **OMTX603-4** | 79 | 5 ± 0.05 |

### EXAMPLE 3: In vivo antitumoral efficacy of the ADCs

Next, OMTX603-2 and 603-3 were tested for their anti-tumoral efficacy in a gastric cancer patient-derived xenograft (PDX) model, ST-02-0318, with high expression of endoglin. Tolerability issues were previously observed with a different anti-FAP eribulin ADC having a DAR of 12, and so OMTX603-4 was not selected for *in vivo* testing for this reason.

In the first experiment, OMTX603-2 was tested at two different weekly i.v doses, 5 and 20mg/kg in comparison to a control vehicle dose and OMTX703 at a weekly i.v. dose of 5mg/kg. After 4 weeks of treatment, OMTX603-2 showed dose-response with tumour growth inhibition (TGI) of 34.72% at 5mg/kg and an even greater TGI (72.83%) at 20mg/kg (Figure 2A). No tolerability issues were observed when monitoring animal weight (Figure 2B).

TGI was calculated according to the following formula: TGI (%) = [1 - (T*ᵢ* - T₀)/ (V*ᵢ* - V₀)] × 100, where Tᵢ is the average tumor volume of a treatment group on a given day, T₀ is the average tumor volume of the treatment group on the day of the start of treatment, Vᵢ is the average tumor volume of the vehicle control group on the same day as Tᵢ, and V₀ is the average tumor volume of the vehicle group at the start of treatment.

In the second experiment, OMTX603-3 was tested at two different weekly i.v doses, 10 and 20mg/kg in comparison to a vehicle control and another ADC "OMTX103" (see Table 1). OMTX103 is an ADC comprising an endoglin-targeting antibody OMTX003 conjugated to a different linker and payload (MMAE). OMTX603-3 showed dose response in this experiment. Anti-tumor efficacy was observed at both doses, although this was increased at 20mg/kg, reaching complete tumor regression and no regrowth up to 42 days after treatment start (Figure 2C). At both concentrations, anti-tumor efficacy was at least comparable to and indeed slightly improved relative to OMTX103. No tolerability issues were observed when monitoring animal weight (Figure 2D).

### Sequences

***Anti-human Endoglin A5-IgG1-HC:*** 
METDTLLLWVLLLWVPGSTG
EVQLLESGGGLVQPGGSLRLSCAASGFTFSSYAMSWVRQAPGKGLEWVSAIYGSDGDTTYADSVKGRF
TISRDNSKNTLYLQMNSLRAEDTAVYYCARVFYTAGFDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPPVAGPSVFLFPPKPKDTLMISRTPEVTCVVVDVSHEDPEVKFN
WYVDGVEVHNAKTKPREEQYNSTYRVVSVLTVLHQDWLNGKEYKCKVSNKGLPSSIEKTISKAKGQPRE
PQVYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO: 1)
***Anti-human Endoglin A5-IgG1-LC:*** 
METDTLLLWVLLLWVPGSTG
DIELTQSPSSLSASVGDRVTITCRASQSISSSLNWYQQKPGKAPKLLIYAASSLQSGVPSRFSGSGSGTD
FTLTISSLQPEDFATYYCQQAPAKPPTFGQGTKLEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC (SEQ ID NO: 2)
A5-IgG1-HC - without signal sequence: 
EVQLLESGGGLVQPGGSLRLSCAASGFTFSSYAMSWVRQAPGKGLEWVSAIYGSDGDTTYADSVKGRF
TISRDNSKNTLYLQMNSLRAEDTAVYYCARVFYTAGFDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPPVAGPSVFLFPPKPKDTLMISRTPEVTCVVVDVSHEDPEVKFN
WYVDGVEVHNAKTKPREEQYNSTYRVVSVLTVLHQDWLNGKEYKCKVSNKGLPSSIEKTISKAKGQPRE
PQVYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO: 3)
A5-lgG1-LC - without signal sequence: 
DIELTQSPSSLSASVGDRVTITCRASQSISSSLNWYQQKPGKAPKLLIYAASSLQSGVPSRFSGSGSGTD
FTLTISSLQPEDFATYYCQQAPAKPPTFGQGTKLEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC (SEQ ID NO: 4)
A5-VH: 
EVQLLESGGGLVQPGGSLRLSCAASGFTFSSYAMSWVRQAPGKGLEWVSAIYGSDGDTTYADSVKGRF
TISRDNSKNTLYLQMNSLRAEDTAVYYCARVFYTAGFDYWGQGTLVTVSS (SEQ ID NO: 5)
A5-VL: 
DIELTQSPSSLSASVGDRVTITCRASQSISSSLNWYQQKPGKAPKLLIYAASSLQSGVPSRFSGSGSGTD
FTLTISSLQPEDFATYYCQQAPAKPPTFGQGTKLEIKR (SEQ ID NO: 6)
A5-CDRH1:
   SYAMS (SEQ ID NO: 7)
A5-CDRH2:
   AIYGSDGDTTY (SEQ ID NO: 8)
A5-CDRH3:
   VFYTAGFDY (SEQ ID NO: 9)
A5-CDRL1:
   RASQSISSSLN (SEQ ID NO: 10)
A5-CDRL2:
   AASSLQS (SEQ ID NO: 11)
A5-CDRL3:
   QQAPAKPPT (SEQ ID NO: 12)
***Anti-murine Endoglin mE12-IgG1-HC:*** 
METDTLLLWVLLLWVPGSTG
EVQLVESGGGVVQPGRSLRLSCAASGFTFSSYGMHWVRQAPGKGLVWVSRINSDGSSTSYADSVKGR
FTISRDNSKNTLYLQMNSLRAEDTAVYYCARATGTWVMSWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPPVAGPSVFLFPPKPKDTLMISRTPEVTCVVVDVSHEDPEVKFN
WYVDGVEVHNAKTKPREEQYNSTYRVVSVLTVLHQDWLNGKEYKCKVSNKGLPSSIEKTISKAKGQPRE
PQVYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO: 13)
***Anti-murine Endoglin mE12-IgG1-LC:*** 
METDTLLLWVLLLWVPGSTG
SSELIQDPAVSVALGQTVRITCQGDSLRSYYASWYQQKPGQAPVLVIYGKNNRPSGIPDRFSGSSSGNT
ASLTITGAQAEDEADYYCNSRDSSGTVFGGGTKLTVLGQPKANPTVTLFPPSSEELQANKATLVCLISDFY
PGAVTVAWKADGSPVKAGVETTKPSKQSNNKYAASSYLSLTPEQWKSHRSYSCQVTHEGSTVEKTVAP
TECS (SEQ ID NO: 14)
mE12-IgG1-HC - without signal sequence: 
EVQLVESGGGVVQPGRSLRLSCAASGFTFSSYGMHWVRQAPGKGLVWVSRINSDGSSTSYADSVKGR
FTISRDNSKNTLYLQMNSLRAEDTAVYYCARATGTWVMSWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPPVAGPSVFLFPPKPKDTLMISRTPEVTCVVVDVSHEDPEVKFN
WYVDGVEVHNAKTKPREEQYNSTYRVVSVLTVLHQDWLNGKEYKCKVSNKGLPSSIEKTISKAKGQPRE
PQVYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO: 15)
mE12-IgG1-LC - without signal sequence: 
SSELIQDPAVSVALGQTVRITCQGDSLRSYYASWYQQKPGQAPVLVIYGKNNRPSGIPDRFSGSSSGNT
ASLTITGAQAEDEADYYCNSRDSSGTVFGGGTKLTVLGQPKANPTVTLFPPSSEELQANKATLVCLISDFY
PGAVTVAWKADGSPVKAGVETTKPSKQSNNKYAASSYLSLTPEQWKSHRSYSCQVTHEGSTVEKTVAP
TECS (SEQ ID NO: 16)
mE12-VH: 
EVQLVESGGGVVQPGRSLRLSCAASGFTFSSYGMHWVRQAPGKGLVWVSRINSDGSSTSYADSVKGR
FTISRDNSKNTLYLQMNSLRAEDTAVYYCARATGTWVMSWGQGTLVTVSS (SEQ ID NO: 17)
mE12-VL: 
SSELIQDPAVSVALGQTVRITCQGDSLRSYYASWYQQKPGQAPVLVIYGKNNRPSGIPDRFSGSSSGNT
ASLTITGAQAEDEADYYCNSRDSSGTVFGGGTKLTVLG (SEQ ID NO: 18)
mE12-CDRH1:
   SYGMH (SEQ ID NO: 19)
mE12-CDRH2:
   RINSDGSSTSYADSVKG (SEQ ID NO: 20)
mE12-CDRH3:
   ATGTWVMS (SEQ ID NO: 21)
mE12-CDRL1:
   QGDSLRSYYAS (SEQ ID NO: 22)
mE12-CDRL2:
   GKNNRPS (SEQ ID NO: 23)
mE12-CDRL3:
   NSRDSSGTV (SEQ ID NO: 24)
Human endoglin 
MDRGTLPLAVALLLASCSLSPTSLAETVHCDLQPVGPERGEVTYTTSQVSKGCVAQAPNA
ILEVHVLFLEFPTGPSQLELTLQASKQNGTWPREVLLVLSVNSSVFLHLQALGIPLHLAY
NSSLVTFQEPPGVNTTELPSFPKTQILEWAAERGPITSAAELNDPQSILLRLGQAQGSLS
FCMLEASQDMGRTLEWRPRTPALVRGCHLEGVAGHKEAHILRVLPGHSAGPRTVTVKVEL
SCAPGDLDAVLILQGPPYVSWLIDANHNMQIWTTGEYSFKIFPEKNIRGFKLPDTPQGLL
GEARMLNASIVASFVELPLASIVSLHASSCGGRLQTSPAPIQTTPPKDTCSPELLMSLIQ
TKCADDAMTLVLKKELVAHLKCTITGLTFWDPSCEAEDRGDKFVLRSAYSSCGMQVSASM
ISNEAVVNILSSSSPQRKKVHCLNMDSLSFQLGLYLSPHFLQASNTIEPGQQSFVQVRVS
PSVSEFLLQLDSCHLDLGPEGGTVELIQGRAAKGNCVSLLSPSPEGDPRFSFLLHFYTVP
IPKTGTLSCTVALRPKTGSQDQEVHRTVFMRLNIISPDLSGCTSKGLVLPAVLGITFGAF
LIGALLTAALWYIYSHTRSPSKREPVVAVAAPASSESSSTNHSIGSTQSTPCSTSS (SEQ ID NO: 25)
Murine endoglin

MDRGVLPLPITLLFVIYSFVPTTGLAERVGCDLQPVDPTRGEVTFTTSQVSEGCVAQAAN
AVREVHVLFLDFPGMLSHLELTLQASKQNGTETQEVFLVLVSNKNVFVKFQAPEIPLHLA
YDSSLVIFQGQPRVNITVLPSLTSRKQILDWAATKGAITSIAALDDPQSIVLQLGQDPKA
PFLCLPEAHKDMGATLEWQPRAQTPVQSCRLEGVSGHKEAYILRILPGSEAGPRTVTVMM
ELSCTSGDAILILHGPPYVSWFIDINHSMQILTTGEYSVKIFPGSKVKGVELPDTPQGLI
AEARKLNASIVTSFVELPLVSNVSLRASSCGGVFQTTPAPVVTTPPKDTCSPVLLMSLIQ
PKCGNQVMTLALNKKHVQTLQCTITGLTFWDSSCQAEDTDDHLVLSSAYSSCGMKVTAHV
VSNEVIISFPSGSPPLRKKVQCIDMDSLSFQLGLYLSPHFLQASNTIELGQQAFVQVSVS
PLTSEVTVQLDSCHLDLGPEGDMVELIQSRTAKGSCVTLLSPSPEGDPRFSFLLRVYMVP
TPTAGTLSCNLALRPSTLSQEVYKTVSMRLNIVSPDLSGKGLVLPSVLGITFGAFLIGAL
LTAALWYIYSHTRGPSKREPVVAVAAPASSESSSTNHSIGSTQSTPCSTSSMA (SEQ ID NO: 26)

### References

A number of publications are cited above in order to more fully describe and disclose the invention and the state of the art to which the invention pertains. Full citations for these references are provided below. The entirety of each of these references is incorporated herein.
1. Weinberg, R.A., et al., Garland science, Taylor & Francis Group LLC, New York, NY, USA, 2007
2. Nieman, K.M., et al., Nat. Med., 2011, 17: 1498-1503
3. Joyce, J.A., et al., Nat. Rev. Cancer, 2009, 9: 239-252
4. Hanahan, D., et al., Cancer Cell, 2012, 21: 309-322
5. Gupta, G.P., et al., Cell, 2006: 127: 679-695
6. Valastyan, S., et al., Cell, 2011, 147: 275-292
7. Kalluri, R., Nat. Rev. Cancer, 2006, 6: 392-401
8. Pietras, K., et al., Exp. Cell Res., 2010, 316: 1324-1331
9. Orimo, A., et al., Cell, 2005, 121: 335-348
10. Erez, N., et al., Cancer Cell, 2010, 17: 135-147
11. Olumi, A.F., et al., Cancer Res., 1999, 59: 5002-5011
12. Yang, G., et al., Proc. Natl. Acad. Sci. USA, 2006, 103: 16472-16477
13. Hwang, R.F., et al., Cancer Res., 2008, 68: 918-926
14. Hu, M., et al., Proc. Natl. Acad. Sci. USA, 2009, 106: 3372-3377
15. Medema, J.P., et al., Nature, 2011, 474: 318-326
16. Malanchi, I., et al., Nature, 2012, 481: 85-89
17. Strell, C., et al., Ups. J. Med. Sci., 2012, 117: 187-195
18. Horimoto, Y., et al., Cell Adhes. Migr., 2012, 6: 193202
19. Meads, M.B, et al., Nat. Rev. Cancer, 2009, 9: 665-674
20. Olive, K.P., et al., Science, 2009, 324: 1457-1461
21. Acharyya, S., et al., Cell, 2012, 150: 165-178
22. Crawford, Y., et al. Cancer Cell, 2009, 15: 21-34
23. Straussman, R., Nature, 2012, 487: 500-504
24.Kumar S., et al., Cancer Res., 1999, 59: 856-861
25.Li C., et al., Int.J. Cancer, 2000, 89: 122-126
26.Völkel T., et al., Bioch. and Bioph. Res. Com., 2004, 317: 515-521
27.Rüger R., et al., J. of Drug Targeting, 2006, 14: 576-582
28.Müller D., et al., J. of Immun. Methods, 2008, 339: 90-98
29.Uneda S., et al., Int. J. Cancer, 2009, 125: 1446-1453
30.Fonsatti E., et al., Cardiovasc. Res., 2010, 86: 12-19
31.Seon B.K., et al., Curr. Drug Deliv, 2011, 8: 135-143
32.Ferreras J.M., et al., Toxins, 2011, 3: 420-441
33.Muñoz R., et al., Cancer Immunol. Immunother., 2012.

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

## Claims

1. An antibody-drug conjugate having the Formula (II), 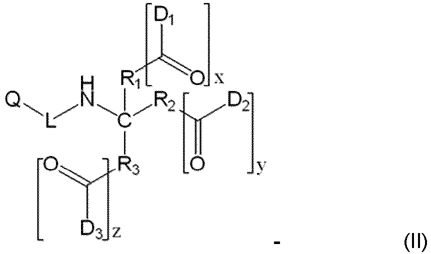
or a pharmaceutically acceptable salt or solvate thereof,
wherein
**Q** is an antibody that selectively binds Endoglin;
L is a linker;
each of D1, D2, and D3 is independently Eribulin;
each of x, y and z is independently 0 or 1, with the provision that at least one of x, y and z is 1;
each of R₁, R₂, and R₃ is independently selected from: a bond, hydrogen, 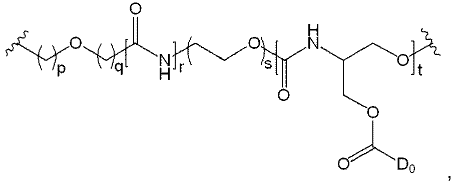 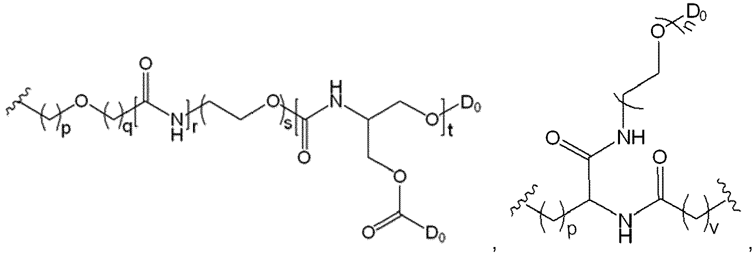 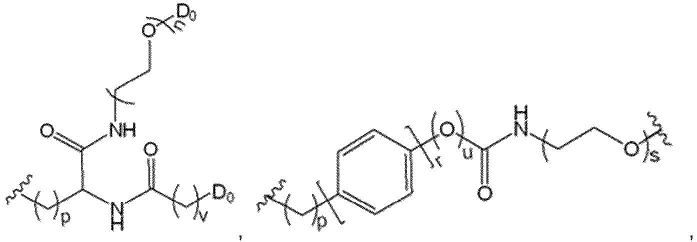 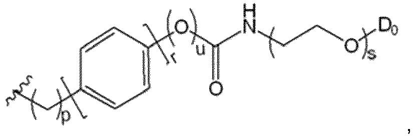 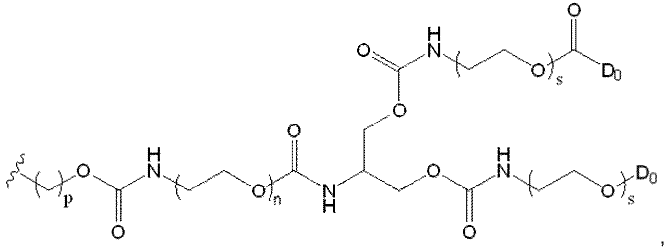 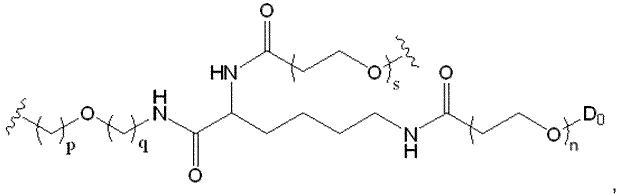
and 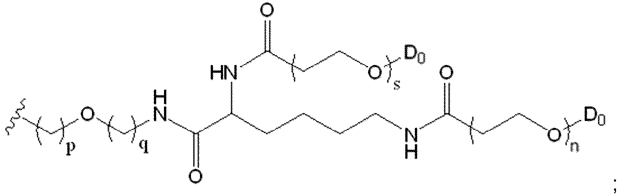
wherein p is 1, 2, or 3; q is 0, 1, or 2; r is 0 or 1; s is an integer from 0 to 37; t is 0 or 1; u is 0 or 1; v is 1, 2, or 3; and n is an integer from 1 to 37; or
x and y are 0 and R₁ and R₂ together form =O and R₃ is 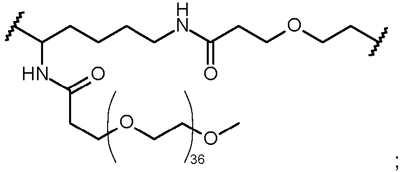
D₀ is methyl, propargyl, or Eribulin;
with a provision that at least one of R₁, R₂, and R₃ is not hydrogen;
if q, r, s, and t are 0, then x+y+z>1;
if x is 0, then R₁ is selected from: hydrogen, 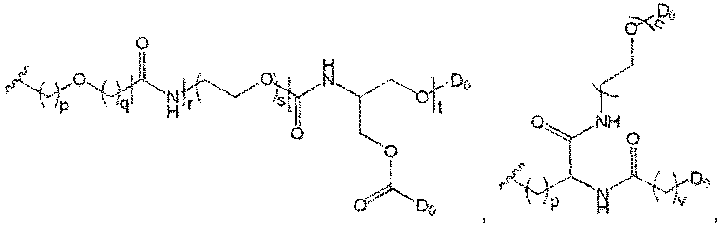 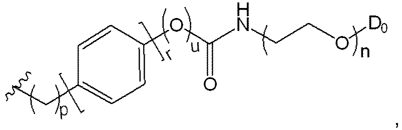 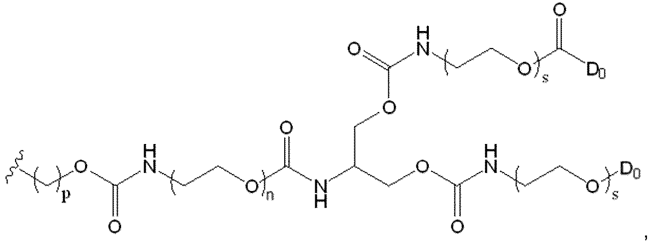 and 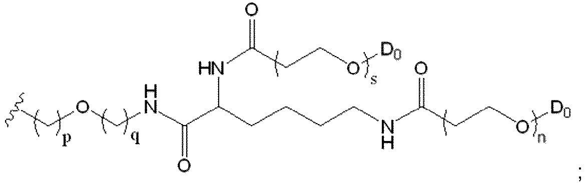
if y is 0, then R₂ is selected from: hydrogen, 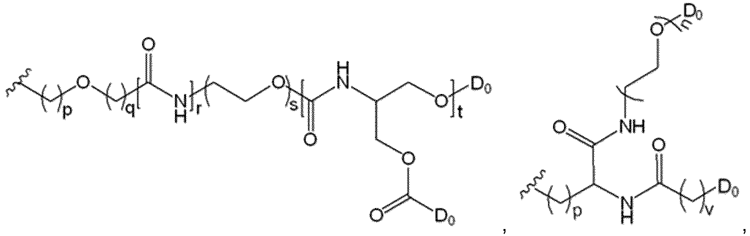 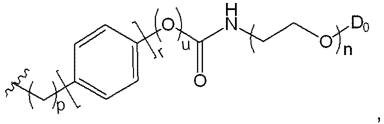 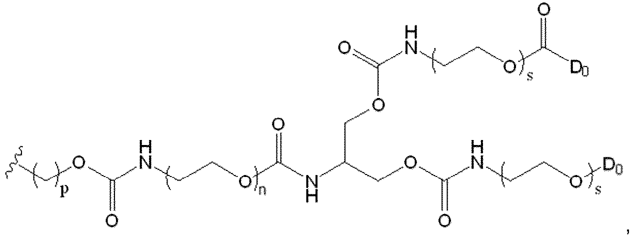 and 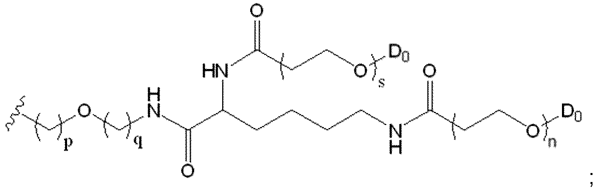
if z is 0, then R₃ is selected from: hydrogen, 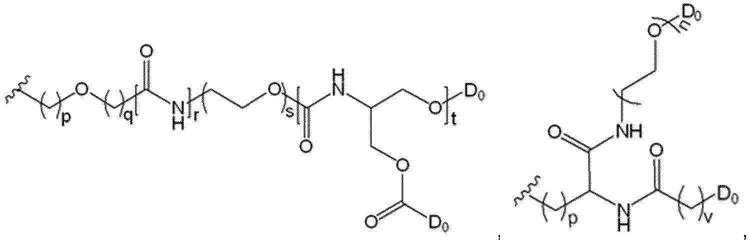 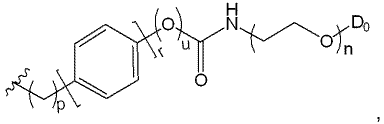 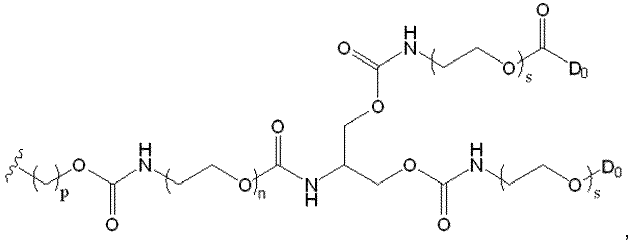 and 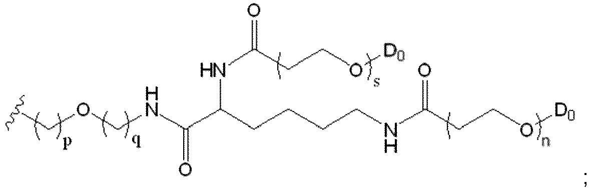
optionally, if x+y+z=1 and each of R₁, R₂, and R₃ independently is 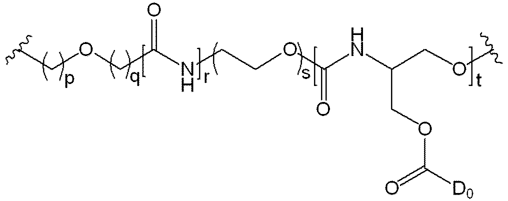 or 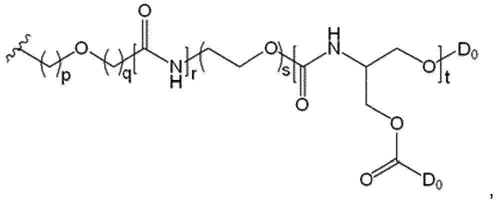 then p is not 1; and
optionally, if each of R₁, R₂, and R₃ independently is 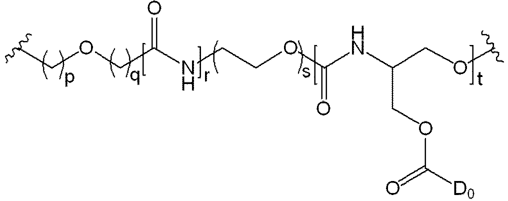 or 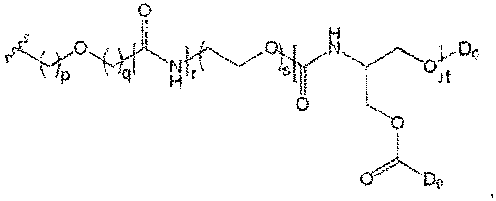 and r, s, and t are each 0,then x+y+z>1.

2. The conjugate according to claim 1, wherein: if x is 0, then R₁ is hydrogen; if y is 0, then R₂ is hydrogen; and if z is 0, then R₃ is hydrogen.

3. The conjugate according to claim 1 or 2, wherein L is selected from the group consisting of: 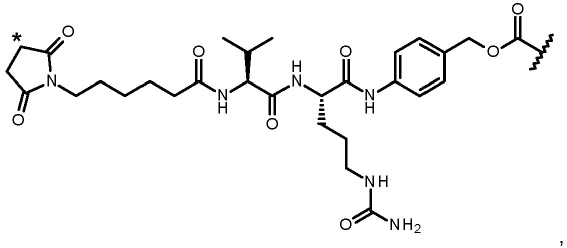 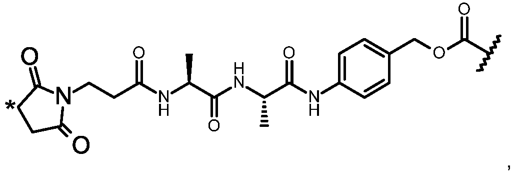 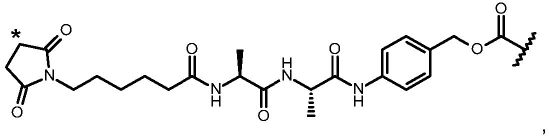 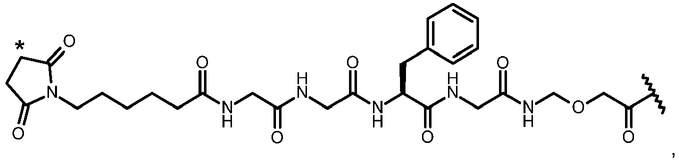 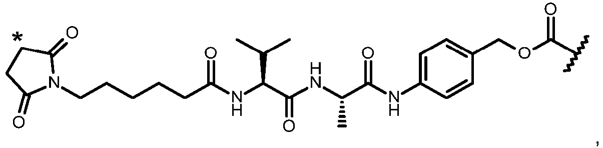 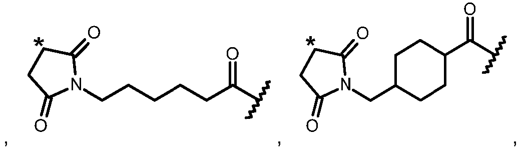 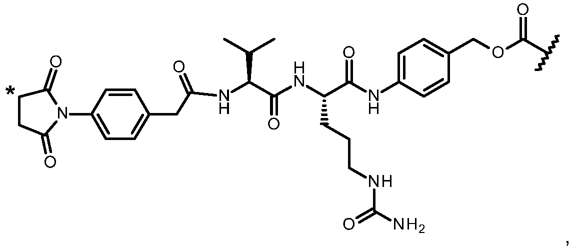 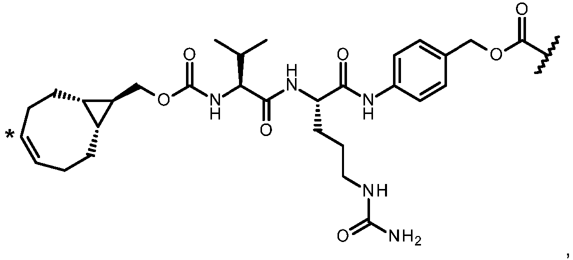 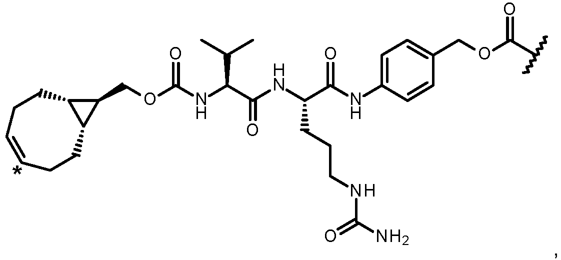 and 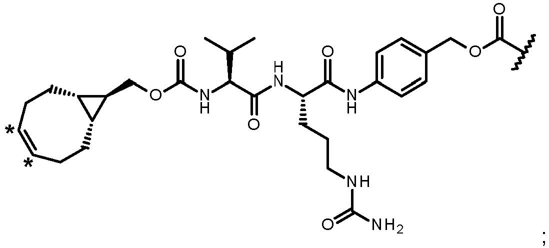 wherein * marks the point of attachment to the antibody Q.

4. The conjugate according to any preceding claim, wherein each of R₁, R₂, and R₃ is independently selected from: a bond, hydrogen, 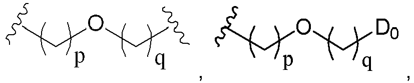 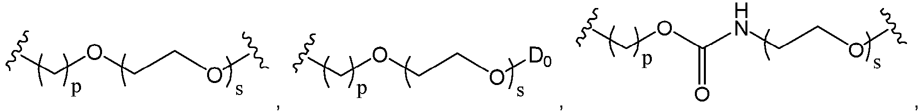 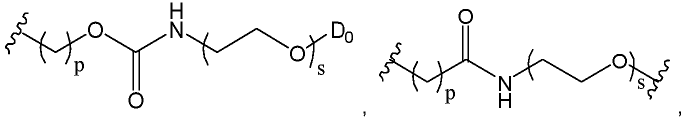 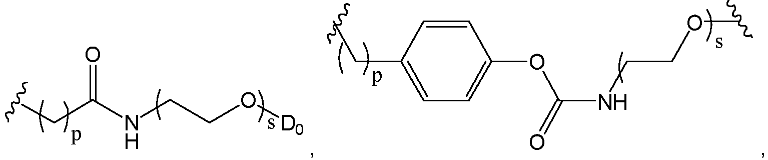 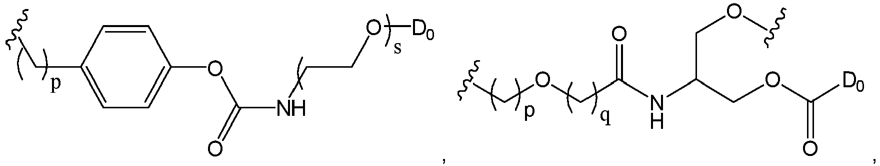 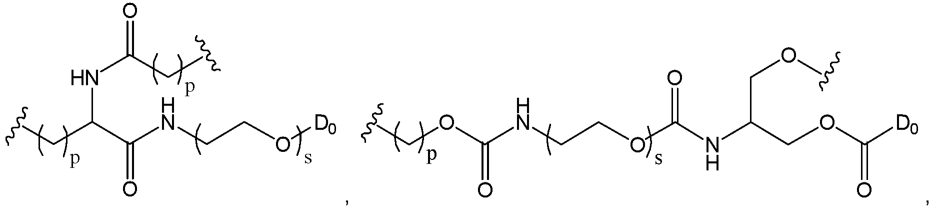 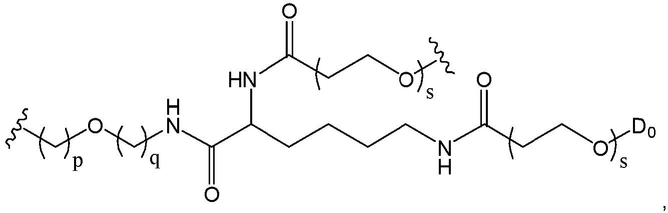 and

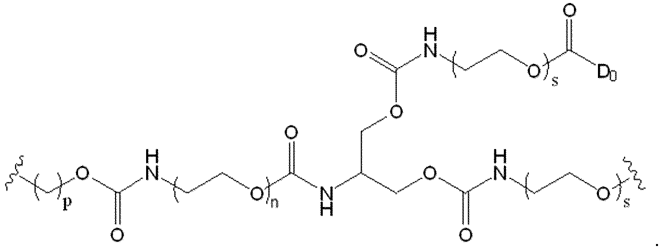

5. The conjugate according to any preceding claim, wherein x+y+z = 1 or 2.

6. The conjugate according to any of the preceding claims, wherein L is

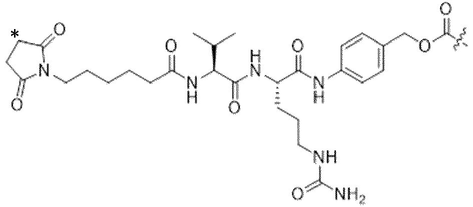

7. The conjugate according to any of the preceding claims, wherein each of R₁, R₂ and R₃ is independently hydrogen,

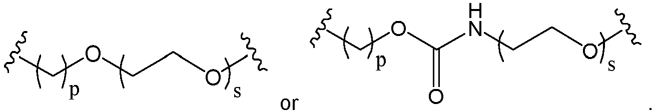

8. The conjugate according to claim 7, wherein s is an integer of from 1 to 4.

9. The conjugate according to claim 7 or claim 8, wherein R₁ and R₂ are hydrogen and R₃ is 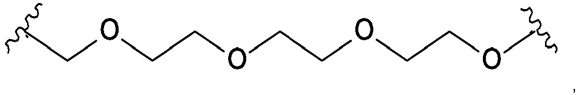 or wherein R₁ is hydrogen and R₂ and R₃ are each 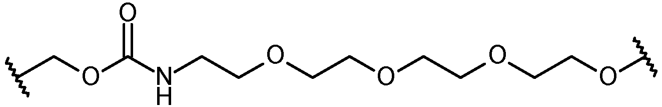 .

10. The conjugate according to any of the preceding claims, wherein x+y+z = 1.

11. The conjugate according to any of claims 1 to 9, wherein the conjugate is: 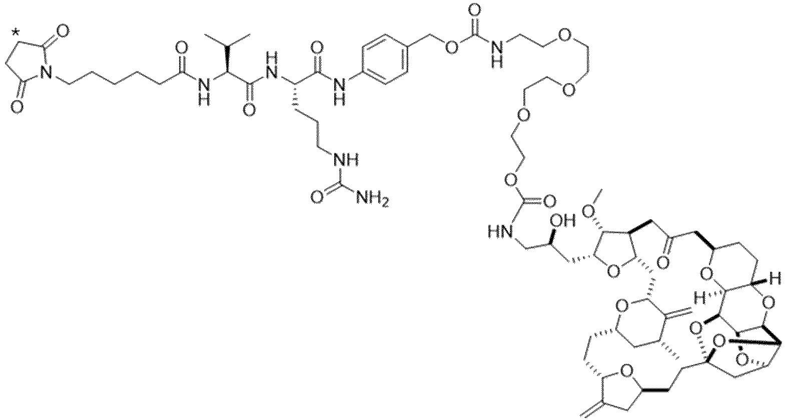 or 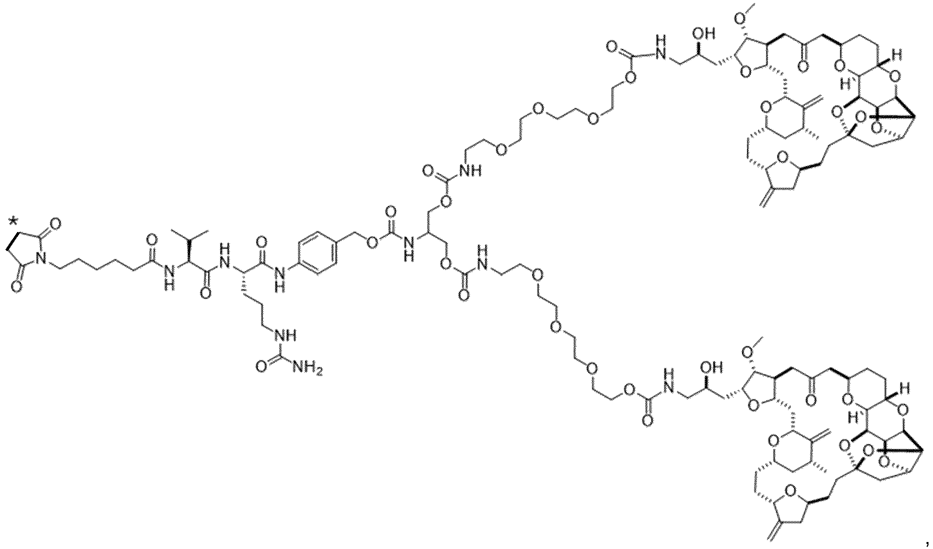 wherein * indicates the point of attachment to the antibody Q.

12. The conjugate according to any one of the preceding claims, wherein the drug antibody ratio (DAR) is from about 3 to 13, preferably wherein the antibody drug ratio is from about 3 to 10, preferably wherein the antibody drug ratio is from about 7 to 9.

13. The conjugate according to claim 12, wherein the conjugate is: 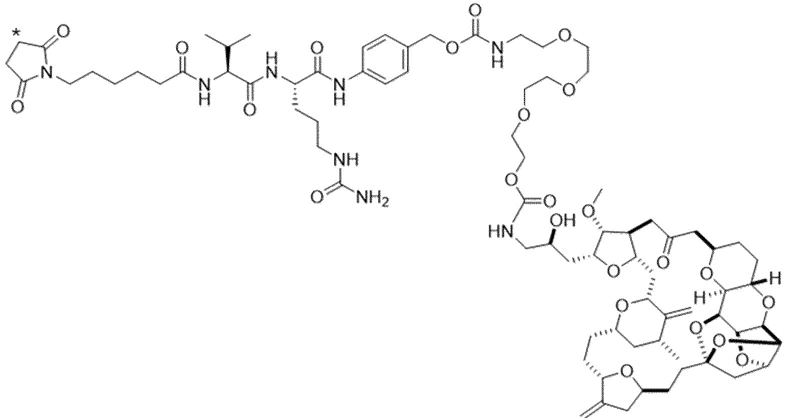 wherein * indicates the point of attachment to the antibody Q, and wherein the antibody drug ratio is from about 7 to 9.

14. The conjugate according to any preceding claim, wherein Q is a monoclonal antibody or binding fragment thereof that selectively binds to an extracellular region of human Endoglin.

15. The conjugate according to claim 14, wherein Q comprises heavy chain complementarity determining regions 1-3 (CDRH1-3) and light chain complementarity determining regions 1-3 (CDRL1-3) having the following amino acid sequences:
(i) CDRH1: SEQ ID NO: 7 or a variant thereof having up to 1 or 2 amino acid substitutions compared with the sequence of SEQ ID NO: 7;
(ii) CDRH2: SEQ ID NO: 8 or a variant thereof having up to 1 or 2 amino acid substitutions compared with the sequence of SEQ ID NO: 8;
(iii) CDRH3: SEQ ID NO: 9 or a variant thereof having up to 1 or 2 amino acid substitutions compared with the sequence of SEQ ID NO: 9;
(iv) CDRL1: SEQ ID NO: 10 or a variant thereof having up to 1 or 2 amino acid substitutions compared with the sequence of SEQ ID NO: 10;
(v) CDRL2: SEQ ID NO: 11 or a variant thereof having up to 1 or 2 amino acid substitutions compared with the sequence of SEQ ID NO: 11; and
(vi) CDRL3: SEQ ID NO: 12 or a variant thereof having up to 1 or 2 amino acid substitutions compared with the sequence of SEQ ID NO: 12; optionally
wherein CDRH1-3 comprise the amino acid sequences of SEQ ID NOS: 7-9, respectively and CDRL1-3 comprise the amino acid sequences of SEQ ID NOS: 10-12, respectively

16. The conjugate according to claims 14 or 15, wherein Q comprises:
a) a heavy chain variable region (VH) comprising an amino acid sequence having at least 90%, 95% or 99% sequence identity with the full-length sequence of SEQ ID NO: 5; optionally a heavy chain variable region (VH) comprising the amino acid sequence of SEQ ID NO: 5; and/or
b) comprises a light chain variable region (VL) comprising an amino acid sequence having at least 90%, 95% or 99% sequence identity with the full-length sequence of SEQ ID NO: 6; optionally a light chain variable region (VL) comprising the amino acid sequence of SEQ ID NO: 6.

17. The conjugate according to any one of claims 14 to 16, wherein Q comprises:
a) a heavy chain comprising an amino acid sequence having at least 90%, 95% or 99% sequence identity with the full-length sequence of SEQ ID NO: 3; optionally a heavy chain comprising the amino acid sequence of SEQ ID NO: 3; and/or
b) a light chain comprising an amino acid sequence having at least 90%, 95% or 99% sequence identity with the full-length sequence of SEQ ID NO: 4; optionally a light chain comprising the amino acid sequence of SEQ ID NO: 4.

18. The conjugate according to any one of claims 1 to 17, for use in a method of treating a disease in a mammalian subject.

19. The conjugate according to any one of claims 1 to 17, for use in a method of treating cancer in a mammalian subject, optionally wherein the cancer comprises a solid tumour or a blood cancer, optionally
wherein the cancer comprises gastric cancer, pancreatic cancer, Ewing sarcoma, breast cancer, melanoma, lung cancer, head & neck cancer, ovarian cancer, bladder cancer or colon cancer, further optionally wherein the cancer is an Endoglin-positive cancer,
further optionally wherein the conjugate is for simultaneous, sequential or separate administration with one or more other antitumour drugs,
further optionally wherein the one or more antitumour drugs comprise a cytotoxic chemotherapeutic agent or an anti-angiogenic agent or an immunotherapeutic agent,
further optionally wherein the one or more other antitumour drugs comprise Gemcitabine, Nab-paclitaxel, bevacizumab, itraconazole, carboxyamidotriazole, an anti-PD-1 molecule (for example, nivolumab, tislelizumab, or pembrolizumab) or an anti-PD-L1 molecule.

20. The conjugate for use according to claim 19, wherein the cancer comprises Endoglin-positive gastric cancer, and wherein the method comprises administering the conjugate intravenously at a dose of at least 5 mg/kg, 10 mg/kg, 15 mg/kg or at least 20 mg/kg.
